# EUROPEAN PATENT SPECIFICATION

(11) **EP 1 583 740 B1**
(45) Date of publication and mention of the grant of the patent: **26.05.2010**
(21) Application number: 03814368.1
(22) Date of filing: 22.12.2003
(51) Int. Cl.: C07C 381/12, C07F 5/02, G03F 7/00, G03F 7/004, G03F 7/075, G03H 1/02

(54) **FLUOROARYLSULFONIUM PHOTOACID GENERATORS**
FLUORARYLSULFONIUM FOTOSÄURE ERZEUGENDE VERBINDUNGEN
GENERATEURS PHOTO-ACIDES AU FLUOROARYLSULFONIUM

(30) Priority: 23.12.2002 US 436521 P
(43) Date of publication of application: 12.10.2005
(73) Proprietor: STX Aprilis, Inc., Maynard, MA 01754 (US)
(72) Inventor: KOLB, Eric, S., Acton, MA 01720 (US); CETIN, Erdem, A., San Diego, California 92129 (US); HUTCHINSON, Kirk, D., Sudbury, MA 01776 (US); MINNS, Richard, A., Arlington, MA 02476 (US)
(74) Representative: Stevens, Ian Edward
(86) International application number: PCT/US2003/041175
(87) International publication number: WO 2004/058699

(56) References cited:
- WO-A-94/25507
- US-A- 5 468 902
- PATENT ABSTRACTS OF JAPAN vol. 1998, no. 11, 30 September 1998 (1998-09-30) -& JP 10 152495 A (NIPPON KAYAKU), 9 June 1998 (1998-06-09)
- PATENT ABSTRACTS OF JAPAN vol. 1998, no. 02, 30 January 1998 (1998-01-30) -& JP 09 278814 A (NIPPON KAYAKU), 28 October 1997 (1997-10-28)
- PATENT ABSTRACTS OF JAPAN vol. 1998, no. 02, 30 January 1998 (1998-01-30) -& JP 09 278813 A (NIPPON KAYAKU), 28 October 1997 (1997-10-28)

## Description

### RELATED APPLICATION

This Application claims the benefit of U.S. Provisional Application No. 60/436,521, filed December 23,2002.

### FIELD OF THE INVENTION

This invention is concerned with the photoinitiation of cationic polymerization by a new class of triarylsulfonium photoacid generators (PAGs) that firstly are sensitive to useful emission spectra of commercially available lasers, secondly are substantially thermally stable to premature degradation or polymerization, and thirdly are soluble in siloxane based monomers and polymers, such as those used in recording materials for holographic data storage. These new PAGs can be sensitized, via a dye, to promote or initiate cationic polymerization with visible light at wavelengths greater than about 500 nm, thereby enabling their use with the emission modes of commercially available visible light lasers to effect rapid cationic polymerization for applications including, by way of example, holographic data storage.

More specifically, the goal of this invention is to prepare a new class of triaryl sulfonium photoacid generators ("PAGs") that can be sensitized, via a dye, to promote or initiate cationic polymerization in the visible spectrum at wavelengths greater than about 500 nm.

In particular, this invention relates to a series ofPAGs that are thermally stable, and which can be photo-sensitized via a dye to specifically utilize the emission modes of visible light lasers to effect rapid cationic polymerization. The PAGs of this invention exhibit good solubility in silicone based monomers and polymers and can, by way of example, be used for applications such as holographic data storage.

### BACKGROUND OF THE INVENTION

It is well known that diaryliodonium and triarylsulfonium salts can undergo photo-induced fragmentation to generate aryliodinium or arylsufinium radical-cations species along with other byproducts (Crivello, J.V.: Advances in Polymer Science, 62 1-48 (1984)). These salts can also be photosensitized for response to long wavelength UV and visible light (Crivello et al, J. Polym. Sci. Polym. Chem. Ed., 16, 2441 (1978); Crivello et al., ibid.,17, 1059 (1979); U.S. Pat. No. 4,250,053 (Smith); U.S. Pat. No. 4,069,054 (Smith)). Photosensitization takes place by a redox process with electron transfer from an excited state photosensitizer to the onium salt (Pappas et al, J. Polym. Sci. Polym. Chem. Ed., 22, 77-84 (1984); Crivello et al, J. Polym. Sci. Polym. Chem. Ed., 17, 977 (1979)). In this process the onium salt gets reduced to form a radical species and further decomposes. The photosensitizer, in turn, is oxidized to a radical-cation and it or its decomposition products functions as the cationic initiator. Diaryliodonium and triarylsulfonium salts comprising a non-nucleophilic anion have been shown to have utility as photo-chemically activated initiators of cationic polymerization of monomers and polymers comprising functional groups such as epoxy and vinyl ether. These salts have enjoyed widespread application and commercialization especially in the coatings and ink arenas (Crivello, J.V.: Advances in Polymer Science, 62, 1-48 (1984)).

Typically, diaryliodonium salts are colorless crystalline solids and are thermally stable up to their melting point. In the presence of epoxy monomers, however, thermal decomposition of dissolved or dispersed diaryliodonium salts occurs at lower temperatures. This reduction in decomposition temperature is attributed to the lack of crystalline packing forces in the dissolved or dispersed state. The parent diphenyl iodonium salts generally have poor solubility in organic media and are usually chemically modified to impart structural irregularities to improve solubility.

Triarylsulfonium salts are also colorless crystalline solids, and are substantially more thermally stable than their iodonium counterparts, due primarily to the inherent strength of the CS bond as compared to the CI bond and secondarily, to pπ-dπ interaction between the pyramidal sulfur and the aromatic rings (Crivello, J.V.: Advances in Polymer Science, 62, 1-48 (1984)). The inherent thermal stability of the triarylsulfonium salts makes them ideal candidates for various applications, particularly where shelf life and long-term storage conditions are an issue.

It has been widely observed that triarylsulfonium salts strongly absorb light near 250 nm while the absorption at longer wavelength is comparatively low. In fact the low absorptivity in the 300-450 nm range severely hampers the efficiency of light utilization in the region in which common light sources, such as mercury lamps, provide a substantial portion of their emission (U.S. Pat. No. 4,069,054 (Smith)). Also, the decomposition products of triarylsulfonium salts tend to absorb at or near the same wavelength of the parent compound, effectively suppressing further photolysis of the salt (Crivello, J.V.: Advances in Polymer Science, 62 1-48 (1984); Dektar and Hacker, JACS 112, 6004-6015 1990).

In prior art, various derivatives of triarylsulfonium salts have been prepared and studied in an effort to improve the sensitivity or efficiency of acid production. Generally, it has been shown that the efficiency of acid generation is controlled by the composition or molecular architecture of the organic cation (Dektar and Hacker, J. Am. Chem. Soc. 112, 6004-6015 1990).

Dektar, *et al.* have determined that substituents tend to shift the absorption max to longer wavelength as compared to a triphenyl sulfonium salt. In prior art, however, the impact of substituents on the generation of acid is ambiguous. A comparison of the photochemical decomposition of Ar-S⁺(Ph)₂ salts with a SbF₆ anion, comprising different types of substituents, indicates that groups like F, Cl, and Br tend to behave similarly to the parent triphenyl cation. The addition of electron donating groups that can accommodate a partial positive charge trend toward improved efficiency in acid production, while purely electron withdrawing groups without some resonance stabilization capability substantially reduce the rate of acid formation in this class of material.

It has been shown that the addition of a photo-sensitizer (PS) improves the efficiency of the photochemical response to longer UV irradiation by an electron transfer process. The efficiency of this process is influenced strongly by the instability of the resulting triphenylsulfur radical, where further decomposition prevents or limits back electron transfer which would otherwise compete with initiation by the (PS⁺) (Wang et al J. Am. Chem. Soc. 1999,121, 4364-4368).

Longer wavelength absorbers such as aromatic hydrocarbons, aromatic ketones, heterocyclic compounds, dyes and the like have been used with triarylsulfonium PAGs to effect polymerization. Perylene, for example can sensitize a sulfonium PAG for polymerization up to 475 nm (U.S. Pat. No. 4,026,705; Crivello et al, J. Polym. Sci. Polym. Chem. Ed., 17, 1059-1065 (1979)). However, as the wavelength of the actinic radiation gets longer (lower energy), the photo-response of the initiator system becomes less efficient, such that the rate of initiation is too slow to be utilized for various demanding imaging applications such as holographic data storage or lithographic techniques.

In prior art, studies have shown that the reactivity or acid strength of an onium salt is governed specifically by the ion pair separation of the anion (Crivello, J.V.: Advances in Polymer Science, 62 1-48 (1984)); with BF₄<PF₆<SbF₆ for cation reactivity. Recently, a new classes of onium salts with non-nucleophilic anions, tetrakis(fluoro-aryl)borate, and galate salts were reported (U.S. Pat. No. 5,468,902, U.S. Pat. No. 5,514,728). These salts are reported to be efficient cationic photoinitiators showing improved catalytic activity versus the corresponding salts comprising inorganic anions such as BF₄, PF₆ and SbF₆⁻. Furthermore, it is disclosed that use of a photosensitizer with these PAGs can be used to increase the radiation sensitivity for increased performance attributes, such as shorter photo-cure times, more complete curing and superior light absorption. However, the advantages provided by these new initiator systems for cationic polymerization is described to be in the 200-400 nm wavelength range, below the useful emission wavelengths of lasers useful for holographic data storage.

### SUMMARY OF THE INVENTION

It has now been found that fluoroaryl and chloroaryl sulfonium salts having a fluoroaryl borate counteranion are thermally stable and are efficient photoacid generators that can be sensitized by photosensitizers at long wavelengths (greater than 500 nm) of light with very high efficiency. As shown in Example 5, sulfonium salts of the present invention exhibit rapid sensitization both by broadband (from a low pressure Hg lamp) and green (514 nm) light. In addition, these sulfonium salts are stabile at elevated temperatures when mixed with a miscible diepoxy monomer (e.g., above 180°C as measured by differential scanning calorimetry) (Example 6), which are above the melting point of the salts (Example 7). The melting points of the sulfonium salts with substituted aryl groups are primarily lower than that of the corresponding unsubstituted triphenyl sulfonium tetrakis[3,5-bis(trifluoromethyl)phenyl]borate salt. Lower melting points generally correlate with improved solubility in siloxane compounds such as may be used as monomers and/or binders.

Based on these discoveries, novel PAGs, novel polymerizable media and holographic recording media (HRM) comprising these PAGs and methods of recording with these holographic recording media are disclosed herein.

Unlike polymerizable materials employing sulfonium PAGs of the prior art, that are insensitive to light above 500 nm, the polymerizable media comprising novel PAG compounds of the present invention exhibited ability to provide for rapid and extensive photopolymerization and a low activation threshold at wavelengths above 500 nm consistent with results obtained with classical iodonium salt PAGs with borate anions (Example 8). This result shows that the inventive PAG compounds of the present invention can be used with wavelengths over 500 nm.

Unlike polymerizable media employing sulfonium PAG compounds of prior art, incapable of recording images with light with a wavelength above 500 nm, holographic recording media comprising inventive PAG compounds showed high signal-to-noise ratio for a given number of images (as indicated by high cumulative grating strength) and retained relatively high recording sensitivity even after multiple co-locational holographic images had been recorded (Example 9).

The present invention includes a sulfonium salt represented by Structural Formula (I):

Ar1 Ar2
S+
Ar3

Ar4
Ar7 B- Ar5
Ar6 (I).

Ar₁ is an aryl group, preferably a phenyl group, substituted with one or more fluoroalkyl, or chloro groups. Preferably, Ar₁ is an aryl group (e.g., a phenyl group) substituted with one or more fluoroalkyl groups.

Ar₃ and Ar₃ are independently substituted or unsubstituted aryl groups as set out below.

The groups represented by Ar₄-Ar₇ are independently substituted or unsubstituted aryl groups as set out below.

The present invention also includes a polymerizable medium, where the medium comprises:
a) a sulfonium salt represented by the Structural Formula (V): 
Ar1 Ar2
S+ Y-
Ar3 (V);
 where:
   Ar₁ is an aryl group substituted with one or more fluoroalkyl, fluoro, or chloro groups;
   Ar₂-Ar₃ are independently a substituted or unsubstituted aryl group as defined below ;
   Y⁻ is selected from the group consisting of B(R₈)ₓ(Ar₈)_{y}⁻, BF₄⁻, PF₆⁻, AsF₆⁻, SbF₆⁻, (CF₃SO₂)₃C⁻, (CF₃SO₂)₂N⁻, CF₃SO₃, Ga(C₆F₅)₄, and carboranes;
   each R₈ is independently a substituted or unsubstituted alkyl group as defined below;
   each Ar₈ is independently a substituted or unsubstituted aryl group as defined below; and
   X and y are 0, 1, 2, 3 or 4, wherein the sum of x and y is 4;
b) a "photosensitizer", which in combination with the sulfonium salt produces acid in response to visible light having a wavelength greater than 500 nm; and
c) at least one monomer or oligomer which is capable of undergoing cationic polymerization initiated by the acid.

In one embodiment, the polymerizable medium is a holographic recording medium, which further comprises a binder which is capable of supporting cationic polymerization of the monomer or oligomer.

In another aspect, the present invention is a method of generating acid. This method comprises exposing a sulfonium salt of the present invention to visible light in the presence of a photosensitizer.

A preferred embodiment of the present invention is a method of recording holograms within a holographic recording medium disclosed herein. The method generally comprises the step of passing into the medium a reference beam of coherent actinic radiation and at substantially the same location in the medium simultaneously passing into the medium an object beam of the same coherent actinic radiation, such that the sulfonium salt in combination with the photosensitizer is capable of producing acid upon exposure to the actinic radiation, thereby forming within the medium an interference pattern and thereby recording a hologram within the medium.

Advantages of the present invention include highly active sulfonium salt PAGs that have a high degree of thermal stability. These PAGs can be photosensitized to achieve rapid polymerization of one or more cationic monomers, oligomers, or polymers. Additional advantages of the PAGs of the present invention include excellent solubility in siloxane-based monomers that undergo cationic polymerization (i.e., no solubilization aid or solvent such as methylene chloride is required), and the ability to be sensitized by long-wavelength UV or visible light by use of a sensitizing agent.

### BRIEF DESCRIPTION OF THE DRAWINGS

FIG. 1 is a plot of heat evolved during polymerization of a polymerizable media that uses a PAG compound of the present invention as a function of time. The result, described in Example 8, was obtained by calorimetric analysis.
FIG. 2 is a plot of cumulative grating efficiency as a function of a number of sequentially recorded holograms. The result, described in Example 9, was obtained for a holographic recording media employing a PAG of the present invention.
FIG. 3 is a plot of recording sensitivity as a function of cumulative fluence. The result, described in Example 9, was obtained for a holographic recording media employing a PAG of the present invention.

### DETAILED DESCRIPTION OF THE INVENTION

One embodiment of the present invention is a new class of sulfonium salt PAGs, which can be sensitized by long-wavelength UV or visible light. The disclosed PAGs are chloro, or fluoroalkyl substituted aryl sulfonium cations with non-nucleophilic tetraarylborate counterions, and are represented by Structural Formula (I).

The present invention demonstrates performance attributes differing from the prior art in the use of dye sensitization at wavelengths longer than about 500 nm. The preferred initiators of this invention demonstrate extremely high photosensitivity and can be utilized for dye sensitized cationic polymerization at wavelength of greater than about 500 nm. The high degree of sensitivity to dye photosensitization at wavelengths greater than about 500 nm is a novel feature of this invention. This invention also provides an initiator system exhibiting a uniquely high degree of stability in the pre-exposed or pre-recorded medium, a feature that is important for exceptional shelf life features that, for example, includes but is not limited to substantially improved pre-recording shelf life in holographic recording media.

When Ar₁-Ar₃ are as described in Structural Formula (I), Ar₄ is preferably substituted with one or more fluoro or fluoroalkyl groups. More prererably, Aᵣ₄-Ar₇ are independently an aryl group substituted with one or more fluoro or fluoroalkyl, groups. Phenyl is a preferred aryl group for Ar₄-Ar₇.

In one example, Ar₁ and Ar₄ are each phenyl groups, and one or both are substituted with one or more fluoro or fluoroalkyl groups. Preferably, Ar₁ is substituted with one or more perfluoroalkyl groups (e.g., trifluoromethyl groups at 3-and/or 5-positions of the ring) or is perfluorinated. Even more preferably, Ar₄-Ar₇ are all phenyl groups independently substituted with one or more fluoro or fluoroalkyl groups, to give, for example, perfluorinated or 3,5-bis(trifluoromethyl) substituted phenyl groups.

As discussed above, fluoro and fluoroalkyl are substituents for Ar₁ and Ar₄-Ar₇. A fluoroalkyl group is a straight chain or branched alkyl group, typically from one to four carbon atoms substituted with at least one fluorine atom. Examples include fluoromethyl, difluoromethyl, trifluoromethyl, fluoroethyl, difluoroethyl, trifluoroethyl, tetrafluroethyl, pentafluoroethyl, fluoropropyl, difluoropropyl, trifluoropropyl, tetrafluoropropyl, pentafluoropropyl, hexafluoropropyl, heptafluoropropyl, fluorobutyl, difluorobutyl, trifluorobutyl, tetrafluorobutyl, pentafluorobutyl, hexafluorobutyl, heptafluorobutyl, octafluorobutyl, and nonafluorobutyl, as well as similar analogs of isopropyl, isobutyl, and sec-butyl groupings. Preferably, a fluoroalkyl group is perfluorinated, i.e., all of the hydrogen atoms have been replaced by fluorine atoms. Examples of perfluoroalkyl groups include -CF₃, -CF₂F₃, -CF₂CF₂CF₃, -CF(CF₃)₂, -CF(CF₃)CF₂CF₃, -CF₂CF(CF₃)₂, and - CF₂CF₂CF₂CF₃. A fluoro substituted aryl group represented by Ar₁ and Ar₄-Ar₇ is preferably perfluorinated, i.e., all hydrogen atoms have been replaced with fluorine. Perfluorinated phenyl is one example. Examples of Ar₁ include perfluorophenyl, 3-trifluoromethylphenyl, and 3,5-bis(trifluoromethyl)phenyl. Preferred examples of Ar₄-Ar₇ are perfluorophenyl and 3,5-bis(trifluoromethyl)phenyl, independently selected.

Ar₁ and Ar₄-Ar₇ can optionally be substituted with other groups which 1) do not react under conditions which induce or initiate cationic polymerization of epoxides; 2) do not interfere with acid initiated cationic polymerization of epoxides; 3) and do not interfere with chemical segregation of binder from polymer formed during cationic polymerization of epoxides. Examples of suitable additional substituents for Ar₁ and Ar₄-Ar₇ include, halogens, R₃Si-, C₁-C₄ alkyl groups, and aryl groups. Each R is independently an unsubstituted aliphatic group or an unsubstituted aryl group, preferably an alkyl group or an aryl group.

In the present invention, Ar₂ and Ar₃ can be independently substituted or unsubstituted aryl groups, preferably phenyl groups. The substituents include fluoro, chloro, bromo, alkyl, fluoroalkyl, chloroalkyl, bromoalkyl, perflouroalkyl and percholoralkyl.

Specific examples of a sulfonium salt of the present invention is represented by the Structural Formula (II):

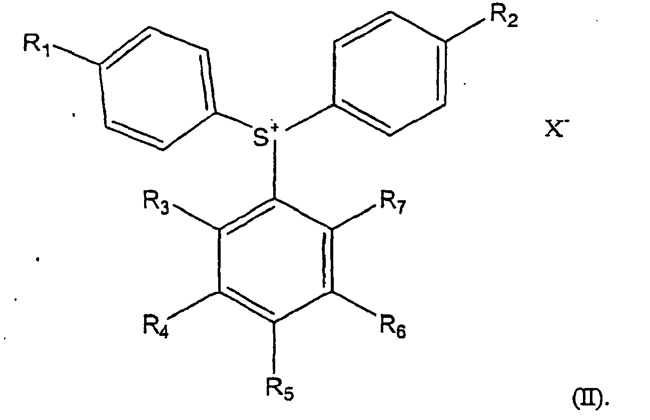

In one example, R₁ and R₁ are methyl; R₃ and R₅-R₇ are H; and R₄ is -CF₃ or -Cl.

In another example, R₁-R₃ and R₅-R₇ are H; and R₄ is -CF₃ or-Cl.

Alternatively, R₁ and R₂ are methyl; R₃, R₅ and R₇ are H; and R₄ and R₆ are -CF₃; or

In yet another example, R₁-R₃, R₅ and R₇ are -H; and R₄ and R₆ are -CF₃.

In each of the above examples, X⁻ is represented by a Structural Formula (III) or (IV):

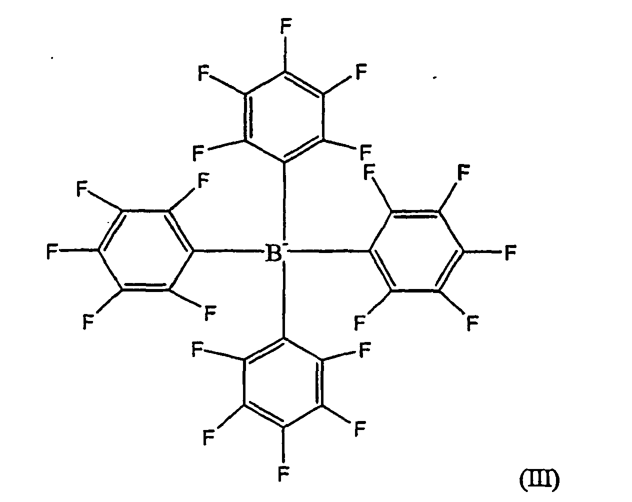

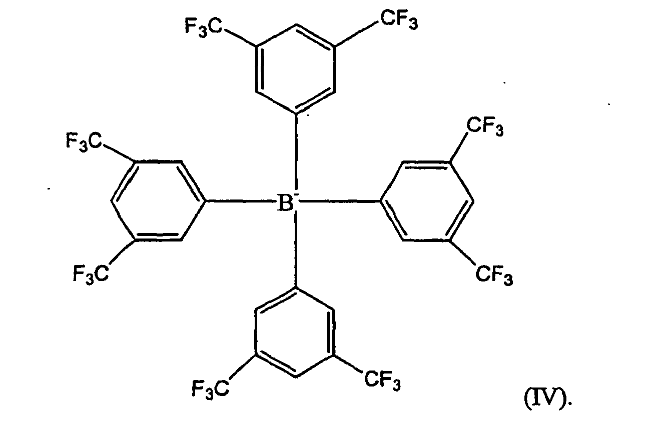

Acid generated by the method of the present invention can be used in polymerizing one or more polymerizable monomers, as is described above. Such polymerizable monomers can form protective, decorative and insulating coatings (e.g., for metal, rubber, plastic, molded parts or films, paper, wood, glass cloth, concrete, ceramics), potting compounds, printing inks, sealants, adhesives, molding compounds, wire insulation, textile coatings, laminates, impregnated tapes, varnishes, and antiadhesive coatings. Acid generated by this method can also be used to etch a substrate or to catalyze or initiate a chemical reaction for application in lithography and/or photo-resist technologies. A particularly advantageous use of this method is to generate acid in only a desired location (e.g., where a laser beam is focused), so as to limit the volume or area in which a chemical reaction catalyzed or initiated by the acid occurs.

Monomers suitable for use in polymerizable media, particularly holographic recording media, typically undergo acid-initiated cationic polymerization (also referred to as "cationic monomers"). Such monomers typically contain one or more vinyl, epoxide, oxetane, cyclic ether, vinyl ether, unsaturated hydrocarbon, lactone, cyclic ester, lactam, cyclic carbonate, cyclic acetal, aldehyde, cyclic sulfide, or cyclosiloxane functional groups, or a combination thereof. 1-Alkenyl ethers, such as vinyl ether or 1-propenyl ether, epoxide functional groups and oxetane groups are common.

Siloxanes substituted with one or more epoxide moieties are commonly used in holographic recording media. A preferred type of epoxy group is a cycloalkene oxide group, especially a cyclohexene oxide group. Siloxane monomers can be difunctional, such as those in which two or more epoxide groupings (e.g., cyclohexene oxide groupings) are linked to an Si-O-Si grouping. These monomers have the advantage of being compatible with the preferred siloxane binders. Exemplary difunctional epoxide monomers are those of Structural Formula (V):

RSi(R¹)₂OSi(R²)₂R (V)

where each group R is, independently, a monovalent epoxy functional group having 2-10 carbon atoms; each group R¹ is a monovalent substituted or unsubstituted C₁₋₁₂ alkyl, C₁₋₁₂ cycloalkyl, aralkyl or aryl group; and each group R² is, independently, R¹, or a monovalent substituted or unsubstituted C₁₋₁₂ alkyl, C₁₋₁₂ cycloalkyl, aralkyl or aryl group. In one particularly useful monomer of this type, each group R is a 2-(3,4-epoxycyclohexyl)ethyl grouping; each grouping R¹ is a methyl group, and each group R² is a methyl group. Monomers of this type are available from Rhodia Corporation, Inc., under the tradename S-200. The preparation of this specific compound is described in, *inter alia,* U.S. Patents Nos. 5,387,698 and 5,442,026.

Siloxane monomers that are suitable for use in polymerizable media can also be polyfunctional. A "polyfunctional" monomer is a compound having at least three groups of the specified functionality, in the present case at least three epoxy groups. The terms "polyfunctional" and "multifunctional" are used interchangeably herein. Polyfunctional monomers have the advantage of being compatible with the preferred siloxane binders and providing for rapid polymerization.

One example of polyfunctional monomers suitable for use in polymerizable media typically has three or four epoxides (preferably cyclohexene oxide) groupings connected by a linker through a Si-O group, i.e., a "siloxane group", to a central Si atom. Alternatively, the epoxides are connected by a linker to a central polysiloxane ring. Examples of such polyfunctional monomers are found in U.S. Publication No. 2002/0065223 and PCT Publication WO 02/19040.

Specific examples of siloxane monomers of this type include the compounds represented by Structural Formulae (VI)-(IX):

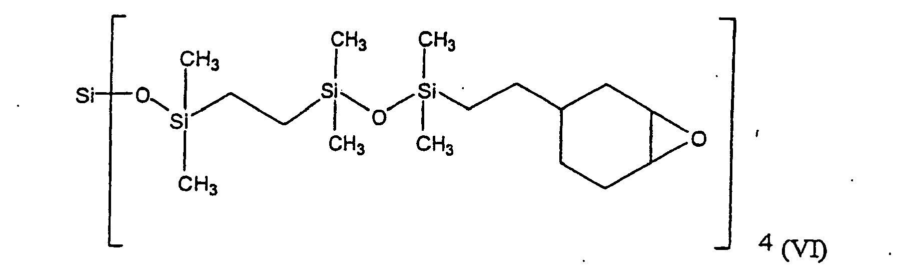

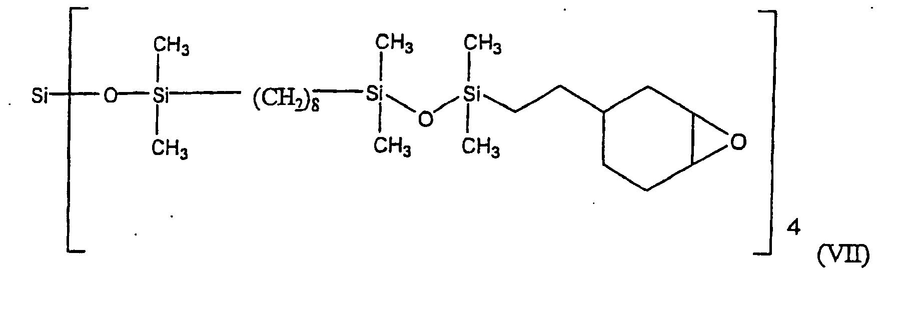

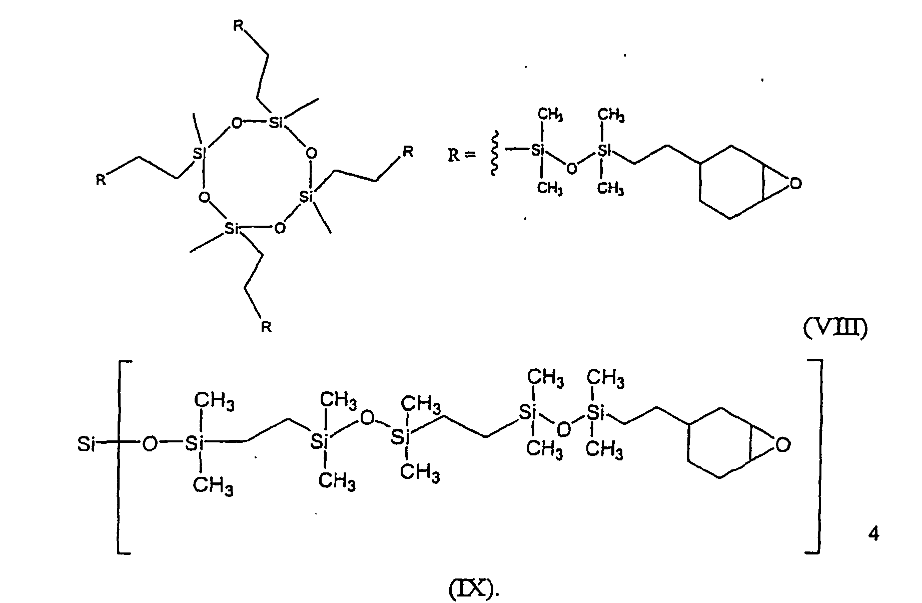

Further description of suitable siloxane monomers can be found in U.S. Publication No. 2002/0068223 and PCT Publication WO 02/19040.

Optionally, the polymerizable medium additionally comprises a second or third monomer that undergoes cationic polymerization or, alternatively, supports cationic polymerization. In one example, monomers that support cationic polymerization can be essentially inert to cationic polymerization. In one example, the second monomer is a vinyl ether comprising one or more alkenyl ether groupings or a propenyl ether comprising one or more propenyl ether groupings. In another example, the second monomer is a siloxane comprising two or more or three or more cyclohexene oxide groups, as described above. Advantageously, the second monomer is a siloxane having at least two cyclohexene oxide groups and the third monomer is a siloxane having at least two cyclohexene oxide groups. The use of additional monomers is described in U.S. Publication No. 2003/0157414.

A binder used in the process and preparation of the present medium should be chosen such that it does not inhibit cationic polymerization of the monomers used (e.g., "supports" cationic polymerization), such that it is miscible with the monomers used, and such that its refractive index is significantly different from that of the polymerized monomer or oligomer (e.g., the refractive index of the binder differs from the refractive index of the polymerized monomer by at least 0.04 and preferably at least 0.09). Binders in this embodiment are required to increase cohesion in said medium, as is generally the case, and are preferably "diffusible", but can be substantially or wholly non-diffusible. Diffusable binders can, by way of example, segregate from the polymerizing monomer(s) or oligomer(s) during holographic recording via diffusion-type motion of the binder component. Non diffusible binders can be a monomer(s) or oligomer(s) that is pre-polymerized to form a moderate to high molecular weight polymeric or copolymer structure that supports cationic polymerization and is a substantially non diffusible component relative to the time scale of diffusion processes during holographic recording events. In general, binders can be inert to the polymerization processes described herein or optionally can polymerize (by cationic, free radical or other suitable polymerization) during one or more polymerization events. Preferably, a binder is inert to the polymerization processes defined herein and, even more preferably, is diffusible.

Preferred binders, which are diffusible and inert to polymerization, for use in holographic recording media are polysiloxanes, due in part to availability of a wide variety of polysiloxanes and the well documented properties of these oligomers and polymers. The physical, optical, and chemical properties of the polysiloxane binder can all be adjusted for optimum performance in the recording medium inclusive of, for example, dynamic range, recording sensitivity, image fidelity, level of light scattering, and data lifetime. The efficiency of holograms produced by the present process in the present medium is markedly dependent upon the particular binder employed. Commonly used binders include poly(methyl phenyl siloxanes) and oligomers thereof. 1,3,5-trimethyl-1,1,3,5,5-pentaphenyltrisiloxane and other pentaphenyltrimethyl siloxanes are examples. Examples are sold by Dow Corning Corporation under the tradename DOW Corning 705 and DOW Corning 710 and have been found to give efficient holograms.

Examples of a diffusible binder having a polymerizable moiety can be found in U.S. Patent No. 6,759,721. This patent also discloses a siloxane polymer having a number of pendant epoxide (cyclohexene oxide) groups. Specifically, the binder was a poly(methylhydrosiloxane) which was hydrosilated with a 90:10 w/w mixture of 2-vinylnaphthalene and 2-vinyl(cyclohex-3-ene oxide).

Examples of a substantially non-diffusible, inert binder can be found in U.S. Patent Nos. 6,103,454 and 6,165,648. Additional examples of a substantially non-diffusible, inert binder can be found in Dhar, et al., Optics Letters, Vol. 24, No.7, p 487-459, 1999 and Hale, et al., Polymer Preprints, 2001, 42 (2), 793. In such examples, the binder is a solid polymer matrix formed in situ from a matrix precursor by a curing step (curing indicating a step of inducing reaction of the precursor to form the polymeric matrix). It is possible for the precursor to be one or more monomers, one or more oligomers, or a mixture of monomer and oligomer. In addition, it is possible for there to be greater than one type of precursor functional group, either on a single precursor molecule or in a group of precursor molecules. In the present invention, examples of precursors that support cationic polymerization are typically polymerizable by free radical or anionic means and include molecules containing styrene, certain substituted styrenes, vinyl naphthalene, certain substituted vinyl naphthalenes and vinyl ethers, which can optionally be mixed with certain co-monomers.

In the absence of any sensitizer, sulfonium salts are typically only sensitive to radiation in the far ultraviolet region, below about 400 nm. However, the use of ultraviolet radiation is inconvenient for the production of holograms because, for a given level of performance, ultraviolet lasers are substantially more expensive than visible lasers. By the addition of various sensitizers, sulfonium salts can be made sensitive to various wavelengths of actinic radiation (e.g., light) to which the salts are substantially inert in the absence of the sensitizer. Such sensitizers include, by way of example, naphthacene derivatives such as tetraphenylnaphthacene and pentacene derivatives such as dialkylbisphenylethynylpentacene. Preferably, the sensitizer is photobleachable so that the visible absorption of the holographic medium decreases during exposure.

Sensitizers advantageously used in the present invention sensitize sulfonium salts, in conjunction with the sensitizer, to produce acid at a wavelength of light longer than 475 nm. Preferably, wavelengths of light are longer than 475 nm and shorter than 800 nm, longer than 500 nm and shorter than 750 nm, or longer than 500 nm and shorter than 550 nm. Even more preferably, a sensitizer in a holographic recording medium is sensitive to light of about 514.5 nm (e.g., from an argon ion laser) or about 532 nm (e.g., from a frequency-doubled Nd:YAG laser).

Examples of sensitizers that are effective in visible light include those represented by Structural Formula (X)-(XV):

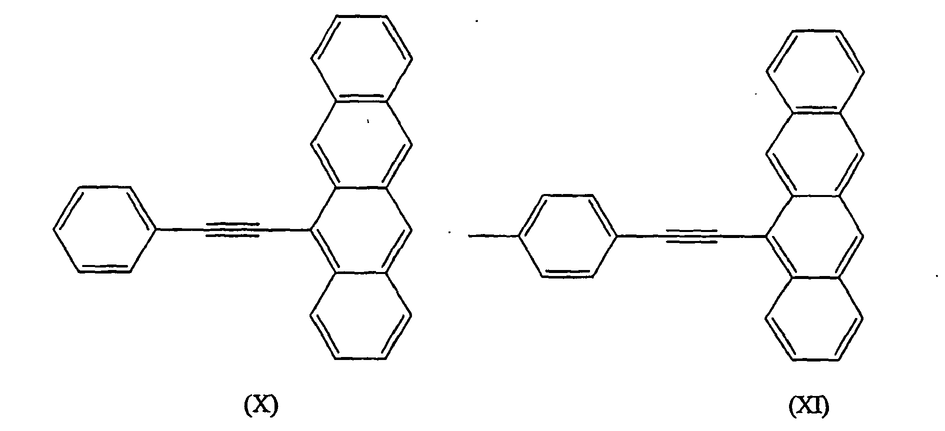

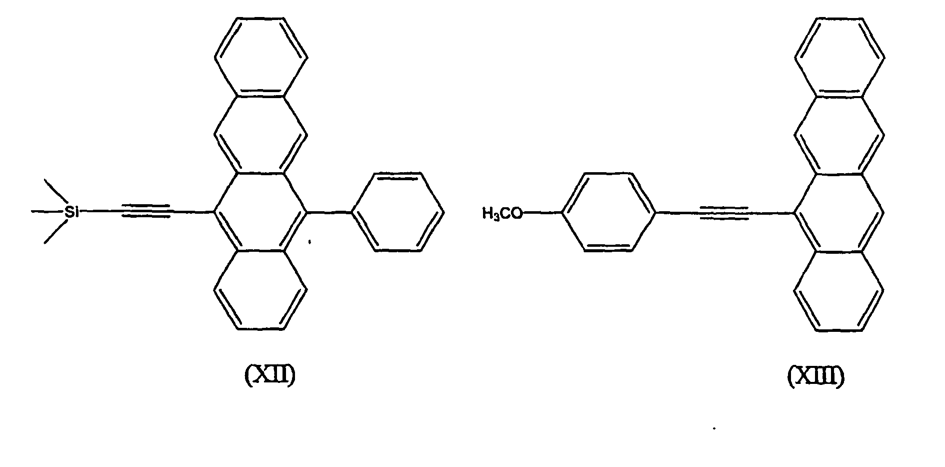

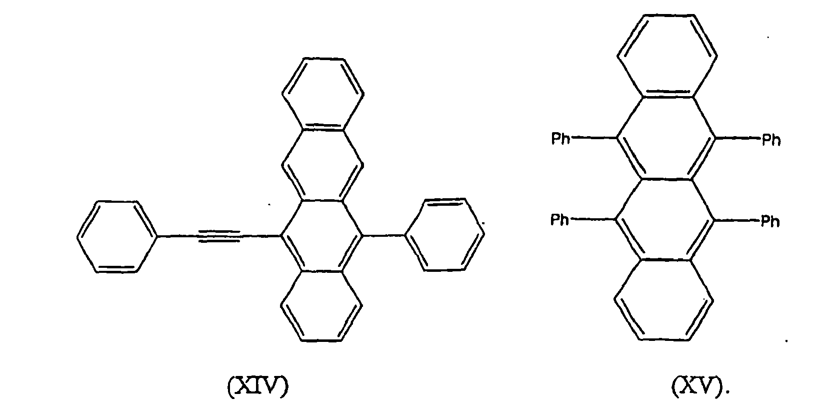

Additional description of suitable dyes for use in the present invention can be found in co-pending U.S. Provisional Application entitled SENSITIZER DYES FOR PHOTOACID GENERATING SYSTEMS, Attorney Docket No. 3174.1008-000, filed on December 23, 2002.

The proportions of sulfonium salt, sensitizer, binder and monomers in holographic recording media of the present invention may vary rather widely, and the optimum proportions for specific components and methods of use can readily be determined empirically by skilled workers. Guidance in selecting suitable proportions is provided in US patent No. 5,759,721.

The solution of monomers with binder can comprise a wide range of compositional ratios, preferably ranging from about 90 parts binder and 10 parts monomer or oligomer (w/w) to about 10 parts binder and 90 parts monomer or oligomer (w/w). It is preferred that the medium comprise from about 0.167 to about 5 parts by weight of the binder per total weight of the monomers. Typically, the medium comprises between about 0.005% and about 0.5% by weight sensitizer, and between about 1.0% and about 10.0% by weight sulfonium salt.

An alkyl group is preferably straight chained or branched with 1 to about 12 carbon atoms, e.g, methyl, methyl, n-propyl, iso-propyl, n.-butyl, sec-butyl, tert-butyl, pentyl, hexyl, heptyl or octyl, or a cycloalkyl group with 3 to about 12 carbon atoms.

Suitable aryl groups for monomers and sulfonium salts of the present invention are those which 1) do not react under conditions which induce or initiate cationic polymerization of epoxides, 2) do not interfere with acid initiated cationic polymerization of epoxides; 3) and do not interfere with chemical segregation of binder from polymer formed during cationic polymerization of epoxides. Examples include, but are not limited to, carbocyclic aromatic groups such as phenyl, naphthyl and biphenyl and heteroaryl groups (e.g., furanyl) and fused polycyclic aromatic ring systems in which a carbocyclic aromatic ring or heteroaryl ring is fused to one or more other heteroaryl rings (e.g., benzofuranyl). Phenyl is a preferred aryl group for Ar₁-Ar₇, substituted as described above.

Substituents for Ar₁-Ar₇ are described above. Suitable substituents for other alkyl and aryl groups (including carbocyclic and heteroaryl) are those which 1) do not react under conditions which induce or initiate cationic polymerization of epoxides; 2) do not interfere with acid initiated cationic polymerization of epoxides; 3) and do not interfere with separation of binder from polymer formed during cationic polymerization of epoxides unless the group comprises an epoxide moiety. The substituents include halogens, R₃Si-, alkyl groups, and aryl groups. Each R is independently an unsubstituted aliphatic group or an unsubstituted aryl group, preferably an alkyl group or an aryl group.

Sulfonium salts of the present invention can be prepared by the procedure of Miller, R. D., Renaldo, A. F., and Ho, H. J. Org. Chem. 1988, 53, 5571-5573 or a modification thereof. In one example, a sulfonium PAG is prepared by reacting two equivalents of a Grignard reagent (e.g., formed from 3-bromobenzotrifluoride) with one equivalent of a diaryl sulfoxide. The reaction is preferably performed in the presence of a catalyst such as trimethylsilyl triflate. The resulting triflate is typically isolated and purified and can be metathesized with the alkali metal salt of a boronate anion (e.g., lithium tetrakis(pentafluorophenyl)borate, sodium tetrakis[3,5-bis(trifluoromethyl)phenyl]borate) to yield the desired product. The product is typically purified by several recrystallizations. A representative synthesis of a sulfonium salt of the present invention is depicted in Scheme 1. The final product, as represented by Structural Formula (XX), is encompassed within Structural Formula (II). Specific conditions for these reactions are provided in Examples 1-3.

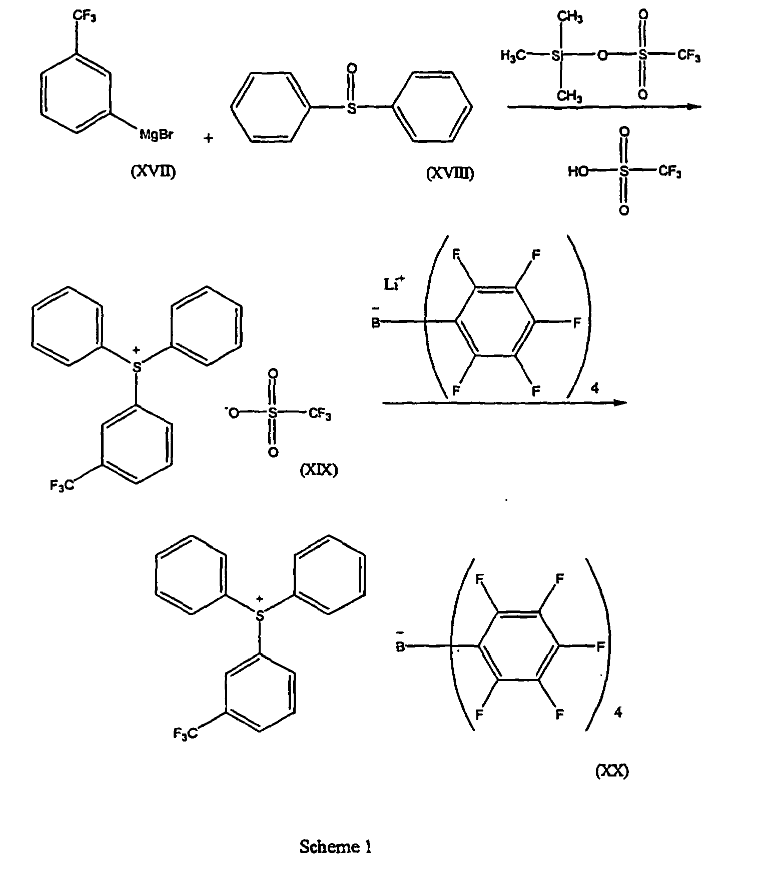

The following examples are not intended to be limiting in any way.

### EXEMPLIFICATION

Examples 1 and 2 are not part of the claimed subject matter, but illustrate the preparation of claimed compounds.

### Example 1

### Preparation of the Grignard reagent

Magnesium turnings (1.2153 g, 50 mmol) were charged into a two-neclced round-bottom flask that was equipped with a magnetic stirrer, reflux condenser, and an addition funnel. Diethyl ether (10 mL) was added to immerse the Mg turnings. The contents were kept under nitrogen. 3-Bromobenzotrifluoride (9.0004 g, 40 mmol) was added dropwise at room temperature. An exothermic reaction started within 2-3 minutes after having added approximately 0.5 mL 3-bromobenzotrifluoride. The reaction contents were diluted with 10 mL of diethyl ether and the rest of 3-bromobenzotrifluoride was added dropwise over 30 min. The reaction was fast and exothermic, and provided a reaction mixture that had a dark brown color. The reaction contents were stirred for 2 hours after the exothermic reaction was over. The yield of Grignard reagent was assumed to be 2.0M (9.9724 g, 40 mmol in 20 mL diethyl ether).

### Example 2

### Preparation of Diphenyl-3-(trifuoromethyl)phenylsulfonium Triflate

As stated above, the triflates were prepared following the procedure of Miller, R D., Renaldo, A. F., and Ho, H., J. Organic Chem., 1988, 53, 5571-73. This procedure was slightly modified to obtain the best yield.

A 100 mL three-necked round-bottom flask was charged with phenyl sulfoxide (4.05 g, 20 mmol) and methylene chloride (40 mL). The reaction vessel was equipped with a magnetic stirrer, an air condenser to which a nitrogen inlet/outlet was mounted, a thermometer, and a suba-seal septum. The reaction contents were cooled to -78°C and treated with trimethylsilyl trifluoromethanesulfonate (5.894 g, 26.5 mmol, 4.8 mL). The addition was completed within 30 min. The reaction mixture was kept at -78°C for 20 min. The contents were warmed to 0°C and kept at 0°C for 30 min. The reaction mixture was cooled to -78°C and previously prepared Grignard solution (2.0M) was slowly added through a cannula. Addition was complete in 30 min. The flask containing the Grignard reagent was washed twice with 5 mL ether and each rinse was cannulated into the reaction flask. The reaction mixture was kept at -78°C for 45 min., then warmed up to 0°C. The contents were kept at 0°C for 45 min. The reaction was quenched with 3% aqueous triflic acid (60 mL). The contents were diluted with diethyl ether (250 mL). The organic layer was separated. The water layer was extracted with 50 mL diethyl ether followed by 50 mL methylene chloride. The combined organic layer was washed with 3% aqueous triflic acid (60 mL) and the organic layer was separated and dried over anhydrous Na₂SO₄ (3.50g). Volatiles were removed via a rotatory evaporator. Solidification of the oily yellow residue was achieved by a rapid dispersion of the oil in in diethyl ether (75 mL). Diethyl ether was slowly added to the oil while stirring it at high speed. High speed stirring continued until the triflate solidified from the ether solution. Solid was isolated by vacuum filtration. The yellow-brown solid was washed with three times 20 mL diethyl ether. The solid was then stirred in 50 mL diethyl ether for 15 min. and then filtered. Yellow-white solid was obtained. The solid was dissolved in 20 mL methylene chloride and ether (~100 mL) was added to reach a cloudy point. The flask was refrigerated. White, shiny crystals were formed. Three crops of crystals were collected. The yield was 48% (4.5990 g, 9.57 mmol).

### Example 3

### Preparation of Diphenyl-3-(trifluoromethyl)phenylsulfonium tetrakis(pentafluorophenyl) borate

Diphenyl-3-(trifluoromethyl)phenylsulfonium triflate (2.2863 g, 4.76 mmol) was dissolved in 20 mL methanol in a 250 mL Erlenmeyer flask that was equipped with a magnetic stirrer. Lithium *tetrakis*(pentafluorophenyl) borate (3.3900 g, 4.94 mmol) was dissolved in 20 mL methanol in a 50 mL Erlenmeyer flask. The lithium *tetrakis*(pentafluorophenyl) borate methanol solution was then added slowly to the methanolic triflate solution at room temperature. The addition was completed within 20 min. The Erlenmeyer flask containing the lithium *tetrakis*(pentafluorophenyl) borate was washed three times with 5.0 mL methanol. Each rinse was combined with the reaction mixture. An additional 10 mL of methanol was used to rinse the walls of the reaction flask. The total amount of methanol used was 65 mL. The reaction contents were stirred at ambient conditions for 45 min. and then water was added dropwise to precipitate the sulfonium salt. The salt precipitated with 2-3 mL water. Additional water (2.0 mL) was added to insure complete precipitation. The salt was collected by filtration, air-dried and purified by flash column chromatography (methylene chloride), followed by recrystallization from methanol/water. The final product was collected by vacuum filtration and dried under high vacuum at room temperature for 18-24 hours. The yield was 71% (3.40 g).

### Example 4

### Preparation of Diphenyl-3-trifluoromethylphenylsulfonium tetrakis[3,5-bis(trifluoromethyl)phenyl] borate

Diphenyl-3-trifluoromethylphenylsulfonium triflate (2.2003 g, 4.58 mmol) was dissolved in 20 mL methanol in a 250 mL Erlenmeyer flask that was equipped with a magnetic stirrer. Sodium *tetrakis*[3,5-bis(trifluoromethyl)phenyl] borate (4.2000 g, 4.74 mmol) was dissolved in 20 mL methanol in a 50 mL Erlenmeyer flask. The procedure of Example 3 was followed. The total volume of methanol was 50 mL. The Erlenmeyer flask containing sodium *tetrakis*[3,5-bis(trifluoromethyl)phenyl] borate was rinsed with 5 mL methanol two times. The rinses were combined with the reaction mixture. The reaction contents were stirred at ambient conditions for 45 min. and then water was added dropwise to precipitate the sulfonium salt. The salt precipitated with 2-3 mL water. Additional water (2.0 mL) was added to insure complete precipitation. The salt was collected by filtration, air-dried and purified by recrystallization from methanol/water. After collection by vaccum filtration the product was dried under high vacuum at room temperature for 18 to 24 hours. The yield was 87% (4.7617 g).

### Example 5

### Broadband and Green Sensitization of Sulfonium PAGs

The sensitization process of a number of sulfonium PAGs was studied by exposing the PAGs to light (either broadband light, from a low pressure mercury lamp, 514 nm or 532 nm green light) and measuring the heat of reaction with a differential scanning calorimeter (DSC). In Tables 2 and 3 below, onset indicates the time at which exothermicity was detected, peak indicates the duration of time for achieving maximum exothermicity, 50% conversion indicates the duration of time for which half of the full extent of polymerization has been achieved, and ΔH is the enthalpy of the polymerization reaction. The enthalpy of reaction was also measured. Improved sulfonium PAGs have shorter onset, peak, and 50% conversion times and larger ΔH values. Also, improved PAGs have ΔH values of equal magnitude in broadband, 514 nm and 532 nm light, and similar rates of exothermicity indicating good sensitization by green light.

Formulations used in the procedure typically comprised the diepoxy monomer, S-200, and the desired triarysulfonium PAG at a loading of∼6 wt%. For sensitized experiments the dye, rubrene, was added to the formulation at a loading of 0.0125 wt%. In a typical PhotoDSC experiment a single drop of formulation dispensed from a micro-syringe with a weight of ∼ 2 mg was placed in the sample pan for evaluation. The formulation to be tested was prepared by charging a clean dried vial with the monomer(s) and the PAG to be evaluated. The mixture was subjected to rapid agitation via a vortex mixer until the PAG has fully dissolved.
For green light sensitized experiments, dye was included in the formulation process via a stock solution in PC1000. All sensitizations with green light used rubrene as the sensitizer.

**Table 1. Compounds tested, where R₁-R₇ refer to substituents of the compound represented by Structural Formula (II).**

| **Compound** | **R₁** | **R₂** | **R₃** | **R₄** | **R₅** | **R₆** | **R₇** |
|---|---|---|---|---|---|---|---|
| **A*** | H | H | H | H | CH₃ | H | H |
| **B** | CH₃ | CH₃ | H | CF₃ | H | H | H |
| **C** | H | H | H | CF3 | H | H | H |
| **D** | H | H | H | Cl | H | H | H |
| **F** | CH₃ | CH₃ | H | CF₃ | H | CF₃ | H |
| **H*** | H | H | H | H | H | H | H |
| **I** | H | H | H | CF₃ | H | CF₃ | H |

| | | | | | | | |
|---|---|---|---|---|---|---|---|
| * comparative cations | | | | | | | |

**Table 2. Broadband Sensitization Data of Sulfonium PAGs**

| **Compound** | **Anion¹** | **Onset (min)** | **Peak (min)** | **-ΔH (J/g)** | **50% Conversion (min)** |
|---|---|---|---|---|---|
| A² | IV | 0.041/0.041 | 0.090/0.093 | 277/278 | 0.112/0.116 |
| B² | IV | 0.040 | 0.300 | 265 | 0.591 |
| B² | III | 0.030 | 0.064 | 313.8 | 0.078 |
| C² | III | 0.040 | 0.433 | 255 | 0.705 |
| C² | IV | 0.043 | 0.480 | 263 | 0.764 |
| D² | III | 0.037 | 0.063 | 314.97 | 0.073 |
| D² | IV | 0.045 | 0.09 | 301.16 | 0.112 |
| F² | III | 0.053 | 0.173 | 293 | 0.300 |
| F² | III | 0.037 | 0.227 | 286 | 0.343 |
| H² | III | 0.039 | 0.078 | 306 | 0.094 |
| I² | III | 0.040 | 0.227 | 293 | 0.378 |

| | | | | | |
|---|---|---|---|---|---|
| ¹ Anion refers to the compound represented by Structural Formula (III) or (IV), as shown above. ² Formulation with S-200. | | | | | |

**Table 3. Green (514 nm or 532 nm) Sensitization Data of Sulfonium PAGs**

| **Compound** | **Anion¹** | **Onset (min)** | **Peak (min)** | **-ΔH (J/g) 514 (532) nm** | **50% Conversion (min)** |
|---|---|---|---|---|---|
| A² | IV | 0.125 | 0.2457 | 278 | 0.419 |
| B² | IV | 0.047 | 0.087 | 312 | 0.098 |
| D² | III | 0.038 | 0.062 | 307.3 | 0.070 |

| | | | | | |
|---|---|---|---|---|---|
| ¹Anion refers to the compound represented by Structural Fonnula (IV) or (V), as shown above. ² Formulation with S-200. | | | | | |

All of the sulfonium PAGs tested exhibited rapid sensitization by broadband light, with 50% of the full extent of polymerization occurring within about 0.1 to about 0.6 minutes. Further, the sulfonium PAGs of the present invention (B, C, D, F and I) were rapidly sensitized by green (514 nm wavelength) light.

### Example 6

### Thermal Differential Scanning Calorimetry (DSC) Data of Sulfonium PAGs

The inherent thermal stability of the triarylsulfonium PAGs was determined using Differential Scanning Calorimetry, DSC. In this experiment a formulation comprising a reactive monomer, PC1000, and the PAG to be tested, was heated at a controlled rate. Under these experimental conditions the temperature at which the PAG begins to decompose and generate acid initiates polymerization of the PC1000. The higher the temperature at the peak of exothermicity for this transition the more thermally stable the PAG is in this formulation. In Table 4, peak indicates the temperature at the peak of exothermicity in °C, peak height is the degree of heat loss in mW at the peak of exothermicity, ΔH is the enthalpy of the polymerization reaction, and heating rate is the rate of increasing temperature in °C/min for the sample.

**Table 4. Thermal DSC Data of Sulfonium PAGs**

| **Compound** | **Anion**¹ | **Peak** **(°C)** | **Peak Height** **(-mW)** | **ΔH** **(J/g)** | **Heating Rate** **(°C/min)** | **Color** |
|---|---|---|---|---|---|---|
| B | IV | 208.70/204.91 | 9.83/ 7.54 | 285/ 290 | 10 | Colorless |
| B | IV | 220.54 | 26.08 | 221 | 30 | Colorless |
| B | IV | 242.88 | 73.79 | 297 | 50 | Colorless |
| C | III | 203.72 | 6.77 | 242.86 | 10 | Colorless |
| C | III | 225.06 | 23.30 | 193.03 | 30 | Colorless |
| C | III | 242.79 | 44.73 | 239.35 | 50 | Colorless |
| C | IV | 194.86 | 10.05 | 350.16 | 10 | Colorless |
| C | IV | 218.08 | 55.77 | 310.71 | 30 | Colorless |
| D | III | 197.5 | 14.0 | 283.62 | 10 | Colorless |
| D | IV | 201.2 | 10.9 | 385.2 | 10 | Colorless |
| F | III | 190 | 10.6 | 316.7 | 10 | Colorless |
| F | III | 212 | 60.1 | 276.4 | 30 | Colorless |
| F | III | 221 | 97.8 | 254.9 | 50 | Colorless |
| H | III | 188 | 5.672 | 109.13 | 10 | Colorless |
| I | III | 182 | 17.68 | 352.6 | 10 | Colorless |
| I | III | 210 | 73.22 | 305.5 | 30 | Colorless |
| I | III | 218 | 136.9 | 293.8 | 50 | Colorless |

| | | | | | | |
|---|---|---|---|---|---|---|
| ¹Anion refers to the compound represented by Structural Formula (IV) or (V), as shown above. | | | | | | |

The PAGs of the present invention exhibited good thermal stability, such that decomposition was not observed until a temperature of at least 180°C. In addition, all sulfonium PAGs remained colorless after the thermal treatment up to 250°C, the limit of the test.

### Example 7

### Melting Point Determination of Sulfonium PAGs by DSC

The melting point of the sulfonium PAGs, listed as peak temperature in °C in Table 5, was determined by differential scanning calorimetry (DSC) at a heating rate of 10 °C/min. Several of the PAGs had dual melting points, indicating two different crystal packing. The enthalpy of melting, ΔH_{M}, is listing using units of both J/g and kJ/mol. All PAGs having more than one entry indicates that the melting point was determined in duplicate or triplicate.

A preferred PAG is stable above the melting point (compare to Example 6). Also, a lower melting power generally correlates with increased solubility in holographic recording media.

**Table 5. Melting point determination of sulfonium PAGs by DSC**

| **Compound** | **Anion¹** | **MW** **(g/mol)** | **Onset** **(°C)** | **Peak** **(°C)** | **-ΔH_{M}** **(J/g)** | **-ΔH_{M}** **(kJ/mol)** | **Heating Rate** **(°C)** |
|---|---|---|---|---|---|---|---|
| A | IV | 1140.61 | 117 | 119 | 42.69 | 48.69 | 10 |
| A | IV | 1140.61 | 117 | 119 | 42.47 | 48.44 | 10 |
| C | IV | 1194.59 | 117 | 119 | 36 | 43.00 | 10 |
| C | IV | 1194.59 | 117 | 118 | 36 | 43.00 | 10 |
| C | III | 1010.41 | 139 | 141 | 41.38 | 41.81 | 10 |
| C | III | 1010.41 | 139 | 141 | 41.45 | 41.88 | 10 |
| D | IV | 1161.03 | 133 | 134 | 40.36 | 46.86 | 10 |
| D | IV | 1161.03 | 133 | 134 | 40.16 | 46.63 | 10 |
| D | III | 976.86 | 120/128 | 122/130 | 36 | 35.17 | 10 |
| D | III | 976.86 | 120/128 | 123/130 | 36 | 35.17 | 10 |
| D | III | 976.86 | 121/129 | 123/130 | 36 | 35.17 | 10 |
| D² | III | 976.86 | 130 | 133 | 35 | 35.17 | 10 |
| F | IV | 1290.64 | 124 | 125 | 39.14 | 50.65 | 10 |
| F | III | 1106.46 | 156 | 158 | 39.73 | 43.96 | 10 |
| H³ | IV | 1126.59 | | 147-149 | | | |
| H³ | III | 942.40 | | 157-159 | | | |
| I⁴ | IV | 1262.60 | 101 | 103 | 33.53 | 42.34 | 10 |
| I | III | 1078.40 | 160 | 162 | 46.38 | 50.02 | 10 |

| | | | | | | | |
|---|---|---|---|---|---|---|---|
| ¹Anion refers to the compound represented by Structural Formula (IV) or (V), as shown above. ²After annealing the sample at 120°C for 15 hours, it exhibited a single melting point. ³Fisher Johns melting point. ⁴After annealing the sample at 90°C for 45 minutes, it exhibited a single melting point. | | | | | | | |

### Example 8.

### Polymerizable media comprising Sulfonium PAG

A sulfoniun salt photoacid generator compound comprising Structural Formula II, wherein R₁, R₂, R₃, R₅, and R₇ are H and R₄ and R₆ are CF₃ groups, and the borate anion of Structural Formula IV was added in an amount 6%w/w of the final recording medium to a difunctional epoxide monomer compound of Structural Formula (V)

RSi(R¹)₂OSi(R²)₂R (V)

where each group R is a 2-(3,4-epoxycyclohexyl)ethyl grouping; each grouping R¹ is a methyl group, and each group R² is a methyl group, available from available from Rhodia Corporation, Inc., under the tradename S-200. This mixture was stirred at room temperature to form a uniform solution. To this solution was added a monofunctional naphthacene dye compound of Structural Formula XIII in an amount 0.03% w/w of the final recording medium, and the resulting mixture was stirred yielding a uniform solution. Convalex-10 (n=1.6325), a polyphenyl ether available from Consolidated Vacuum Corporation, Rochester, NY, was added to this solution as a diffusable binder having high refractive index, and the resulting mixture was stirred overnight at room temperature to yield a uniform and homogenous solution having a compositional ratio of monomer/binder of 70:30 w/w.

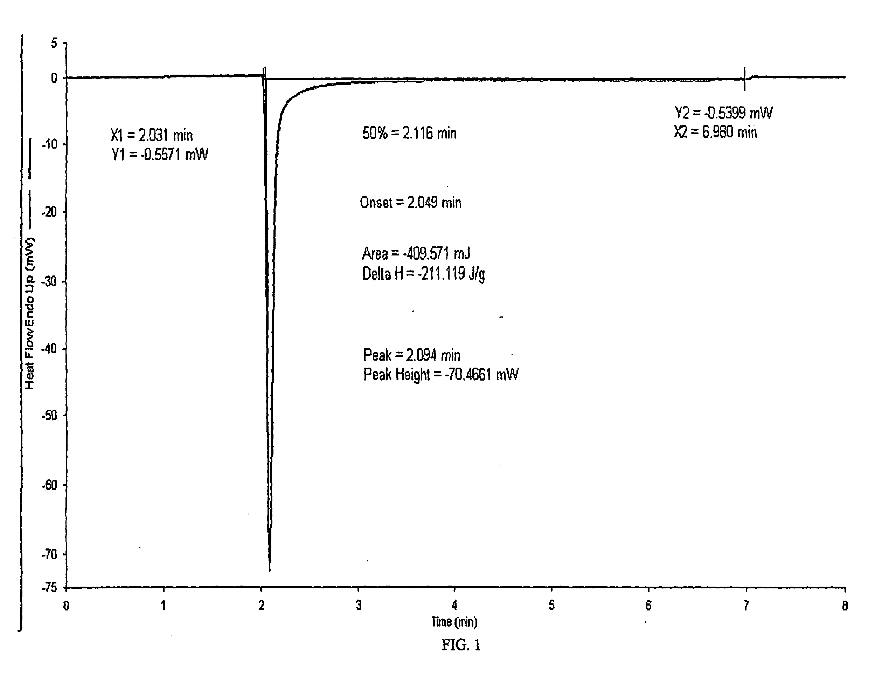

The kinetics and extent of photopolymerization exhibited by this holographic recording medium, shown in Figure 1, was obtained by calorimetric analysis using a Perkin-Elmer DSC-7 Differential Scanning Calorimetry (see Waldman et al., J. Imaging Sci. Technol. 41, (5), pp. 497-514, (1997)) equipped with a DPS-7 photocalorimetric module and a Crystalaser, Inc. diode pumped solid state (DPSS) frequency doubled Nd:YAG laser, emitting at 532 nm, that was coupled into a multimode fiber having a 200 µm core. The peak indicates the time at the peak of exothermicity in minutes, peak height is the degree of heat loss in mW at the peak of exothermicity, ΔH is the enthalpy of the polymerization reaction in J/g, and the 50% time is the time required to achieve 50% of the full extent of polymerization reaction. The shutter control provided for illumination of the sample starting at 2.0 minutes after the calorimetric scan was started, and thus the time to peak is 0.094 minutes and the time to 50% reaction is 0.116 minutes, respectively. These results show that both fast photokinetics of polymerization and a large extent of reaction are achieved in a photopolymerizable medium useful for holographic recording with use of a triarysulfonium salt PAG of this invention at a wavelength of 532 nm.

### Example 9

### Holographic recording with media comprising Sulfonium PAG.

Co-locational slant fringe plane-wave, transmission holograms were recorded in the conventional manner with a frequency doubled Nd:YAG laser (Coheren Vector) emitting at λ=532 nm using two coherent spatially filtered and collimated laser writing beams directed onto the sample with an interbeam angle of 48.6°. The intensities of the two writing beams were equal at the condition of equal semiangles about the normal, and the total incident intensity for recording was 6.45 mW/cm² as measured at the bisecting condition. The sample was mounted onto an optically encoded motorized rotation stage, Model 495 from Newport Corporation, for rotation of ϕ about the perpendicular to the face of the sample in the interaction plane, and this stage was mounted onto an optically encoded motorized rotation statge, 496B from Newport Corporation, for rotation of θ about the vertical axis denoted as the y-axis. Multiplexed co-locational plane-wave transmission holograms were recorded by combining azimuthal and planar-angle multiplexing (see method of Waldman et al., J. Imaging Sci. Technol. 41, (5), pp. 497-514, (1997) ). Azimuthal multiplexing was carried out via rotations of Δϕ about an axis perpendicular to the surface plane of the sample (i.e. z-axis at the condition of equal semiangles for the writing beams) and through the *x-y* center of the imaged area for a specific value of θ, where θ denotes the rotational position of the sample plane about the y-axis, said axis being perpendicular to the interaction plane. Angle multiplexing was carried out in the standard manner by rotation of Δθ which defines *Ω₁ and* Ω*₂*, the external signal and reference writing beam angles, respectively, and thus the grating angle for the plane-wave holograms. Values of ϕ were limited to the range of 0° ≤ ϕ *<* 180° and Δϕ was 1.5°, thus corresponding to 120 co-locational recordings, respectively, for each of the first three grating angle conditions specified by θ having the value of -16°, or -10°, or -4° (counterclockwise rotation) from the bisector condition for the two writing beams. Additionally, a last cycle of 23 holograms was recorded, after a total of 360 were recorded during the first three cycles, by incrementing Δϕ by 8° for θ having the value +7.0° (clockwise rotation). The length of the exposure times was controlled via a direct serial computer interface to a Newport mechancial shutter and a schedule was used that ramped exposure times to longer values in monotonic fashion in accordance with the monotonic decline in recording senstivity that is exhibited by the recording material.

Reconstruction of the 383 co-locationally multiplexed plane-wave gratings was accomplished by utilization of reading beams that corresponded to the recording beams, but with an incident irradiance, measured at normal incidence to the sample, of 4.0 mW/cm². Diffraction intensity data was obtained for all 383 co-locationally recorded holograms, after completion of the recording of the multiplexed holograms, using two Model 818-SL/CM photodiodes and a Model 2835-C dual channel multifunction optical meter from Newport Corporation. Apertures were placed on the face of the photodiode detectors to ensure that diffraction from only one azimuthal angle condition was detected for each Bragg angle that was interrogated. The read angle was tuned to the optimum Bragg condition (i.e. value for maximum diffraction efficiency) for each θ,φ combination used in the multiplexing sequence by rotation of the media about the y-axis for a give value of φ, and the diffraction efficiency was measured at each Δθ angular increment of 0.005° to 0.01° for each θ,φ combination to obtain accurate Bragg detuning profiles for each multiplexed hologram.

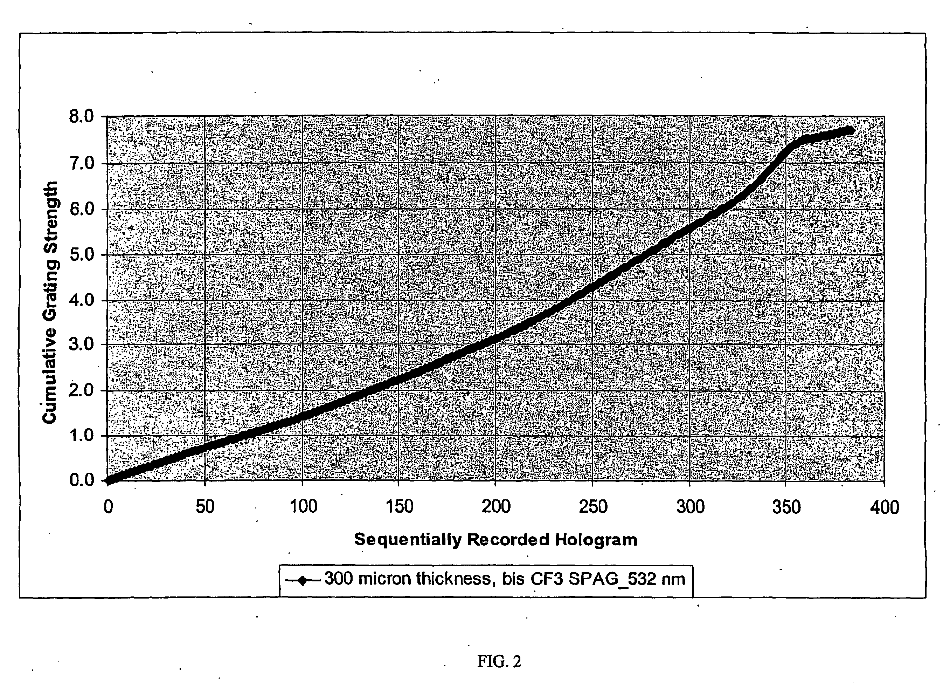

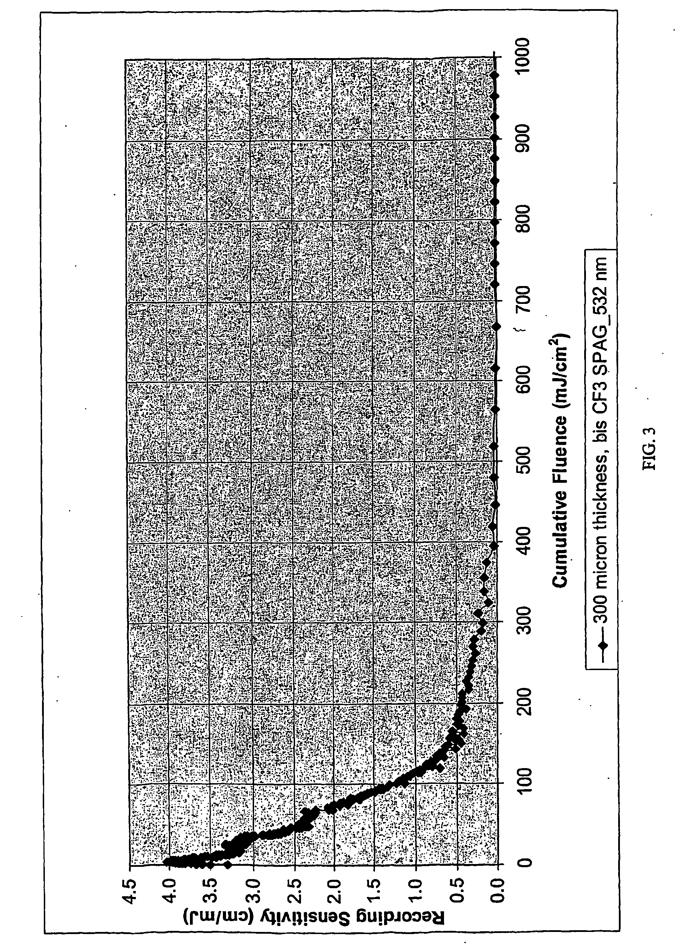

Figure 2 shows growth in cumulative grating strength (Σηᵢ^{0.5}) as determined from the measured values of diffraction efficiency, η*ᵢ*, of each hologram as a function of sequentially recorded hologram number for a coating of the above formulation having 200 micron thickness. The media comprising the triarysulfonium salt PAG of this invention showed evidence of having recorded holograms at the onset of recording (i.e. the first hologram) and thus no threshold was observed for recording at a wavelength of 532 nm. The manifold of cumulative grating strength increased in monotonic fashion from hologram number 1 to hologram number 383 attaining a value of 7.7. The dependence on sequentially recorded hologram number was fairly linear indicative of a reasonably good recording schedule for this type of exposure series. Figure 3 shows recording sensitivity in cm/mJ, as determined from the measured values of diffraction efficiency, η*_{¡}*, of each hologram, as a function of cumulative exposure fluence in mJ/cm². Sensitivity in cm/mJ is calculated in the standard manner as (η*_{¡}^{0.5}*/*Iᵢ***tᵢ*)/T, where *T* is thickness of the recording material, *tᵢ* is the length of the recording time for the ith recording event, and *Iᵢ* is the intensity for the recording event. The recording sensitivity declined with a linear type dependence on cumulative recording fluence from a high of about 4.0 to a low value of about 0.8 8 cm/mJ over a cumulative fluence between 0 and125 mJ/cm², at which point the sensitivity exhibited continued decline but with non linear dependence on cumulative recording fluence. Sensitivity was still about 0.2 cm/mJ after a fluence of 300 mJ/cm², at which point the cumulative grating strength attained a value of about 7.0. These results are similar to those obtained previously with Iodoium salt PAG compounds with borate anions.

While this invention has been particularly shown and described with references to preferred embodiments thereof, it will be understood by those skilled in the art that various changes in form and details may be made therein without departing from the scope of the invention encompassed by the appended claims.

## Claims

1. A sulfonium salt represented by the Structural Formula (I): 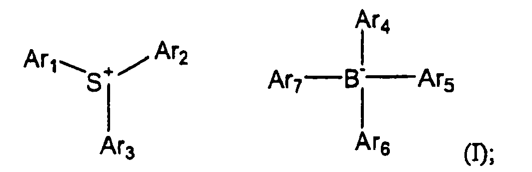 wherein Ar₁ is an aryl group substituted with one or more fluoroalkyl, or chloro groups and Ar₂-Ar₃ are independently an unsubstituted aryl group or an aryl group substituted with fluoro, chloro, bromo, alkyl, fluoroalkyl, chloroalkyl, bromoalkyl, perfluoroalkyle or percholoralkyl, and Ar₄-Ar₇ are independently an unsubstituted aryl group or an aryl group substituted with one or more fluoro or fluoroalkyl groups.

2. The sulfonium salt of Claim 1, wherein Ar₄-Ar₇ are independently an aryl group substituted with one or more fluoro or fluoroalkyl groups.

3. The sulfonium salt of Claim 1 wherein Ar₁ is a phenyl group substituted with one or more fluoroalkyl or perfluoroalkyl groups and Ar₄ is a phenyl groups substituted with one or more fluoro or fluoroalkyl groups.

4. The sulfonium salt of Claim 3 wherein Ar₄-Ar₇ are independently a phenyl group substituted with one or more fluoro or fluoroalkyl groups or perfluorinated or 3,5-bis(trifluoromethyl) substituted phenyl groups.

5. The sulfonium salt of Claim 4 wherein Ar₂-Ar₃ are independently substituted or unsubstituted phenyl groups.

6. The sulfonium salt of Claim 5 wherein the phenyl group represented by Ar₁ is 3-trifluoromethyl substituted or 3,5-bis(trifluromethyl) substituted.

7. A sulfonium salt according to claim 1 represented by the Structural Formula (II): 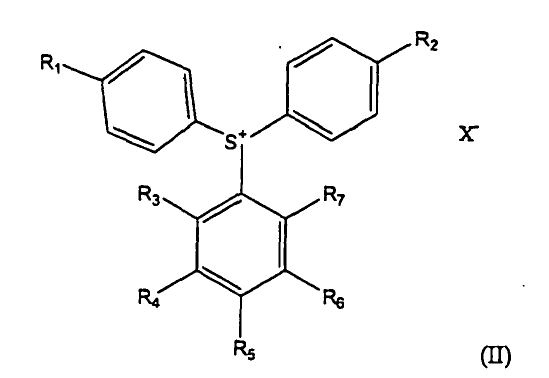 wherein:
R₁ and R₂ are methyl; R₃ and R₅-R₇ are H; and R₄ is -CF₃ or -Cl;
R₁-R₃ and R₅-R₇ are -H; and R₄ is -CF₃ or -Cl;
R₁ and R₂ are methyl; R₃, R₅ and R₇ are -H; and R₄ and R₆ are -CF₃; or
R₁-R₃, R₅ and R₇ are -H; and R₄ and R₆ are -CF₃; and
X⁻ is represented by Structural Formula (III) or (IV): 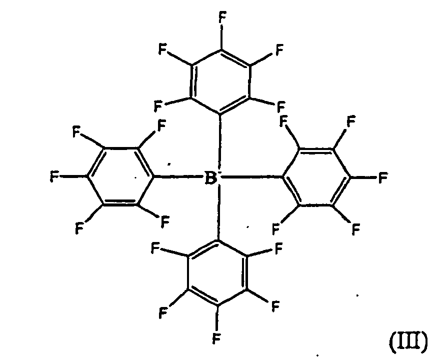 or

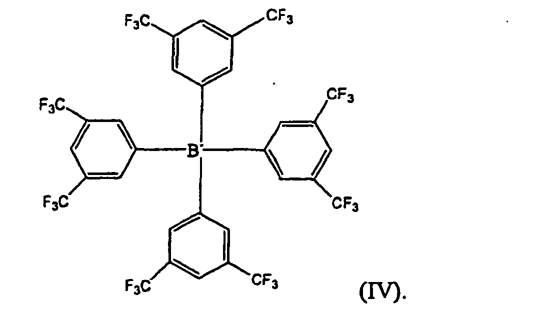

8. A polymerizable medium, wherein said medium comprises:
a) a sulfonium salt represented by the Structural Formula (V): 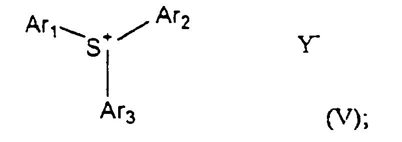 wherein:
Ar₁ is an aryl group substituted with one or more fluoroalkyl, fluoro, or chloro groups;
Ar₂-Ar₃ are independently an unsubstituted aryl group or an aryl group substituted with fluoro, chloro, bromo, alkyl, fluoroalkyl, chloroalkyl, bromoalklyl, perfluoroalkyl or perchloroalkyl;
Y⁻ is selected from the group consisting of B(R₈)ₓ(Ar₈)_{y}⁻ , BF₄⁻, PF₆⁻, AsF₆⁻, SbF₆⁻, (CF₃SO₂)₃C⁻, (CF₃SO₂)₂N⁻, CF₃SO₃⁻, Ga(C₆F₅)₄⁻, and carboranes;
each R₈ is independently an unsubstituted alkyl group or an alkyl group substituted with a halogen, (R)₃Si-, an alkyl group, or an aryl group, wherein each R is independently an unsubstituted aliphatic group or an unsubstituted aryl group;
each Ar₈ is independently an unsubstituted aryl group or an aryl group substituted with a halogen, (R)₃Si-, an alkyl group, or an aryl group, wherein each R is independently an unsubstituted aliphatic group or an unsubstituted aryl group; and
x and y are 0, 1, 2, 3 or 4, wherein the sum of x and y is 4;
b) a "photosensitizer", which sensitizes the sulfonium salt to produce acid in response to visible light having a wavelength greater than 500 nm;
and
c) at least one monomer or oligomer which is capable of undergoing cationic polymerization initiated by the acid produced from the sulfonium salt.

9. The polymerizable medium of Claim 8, wherein the monomer or oligomer which is capable of undergoing cationic polymerization contains one or more epoxide, oxetane, 1-alkenyl ether, cyclic ether, vinyl ether, unsaturated hydrocarbon, lactone, cyclic ester, lactam, cyclic carbonate, cyclic acetal, aldehyde, cyclic sulfide, or cyclosiloxane functional groups, or a combination thereof.

10. The polymerizable medium of Claim 8, wherein said medium comprises a binder.

11. A holographic recording medium, wherein said medium comprises:
a) a sulfonium salt represented by the Structural Formula (V), as defined in Claim 8:
b) a "photosensitizer", which in combination with the sulfonium salt produces acid in response to visible light;
c) a monomer or oligomer which is capable of undergoing cationic polymerization initiated by the acid; and
d) a binder which is capable of supporting cationic polymerization of said monomer or oligomer.

12. The holographic recording medium of Claim 11, wherein the photosensitizer in combination with the sulfonium salt produces acid in response to visible light having a wavelength greater than 500 nm and shorter than 750 nm.

13. The holographic recording medium of Claim 11, wherein the binder is diffusible and inert to polymerization.

14. The holographic recording medium of Claim 11, wherein Ar₁ is an aryl group substituted with one or more fluoroalkyl or fluoro groups, such as an aryl group that is perfluorinated or substituted with one or more perfluoroalkyl groups.

15. The holographic recording medium of Claim 14, wherein Y is represented by the formula B(Ar₄)(Ar₅)(Ar₆)(Ar₇)⁻, wherein Ar₄-Ar₇ are independently an unsubstituted aryl group or an aryl group substituted with one or more fluoro or fluoroalkyl groups.

16. The holographic recording medium of Claim 15, wherein Ar₄-Ar₇ are independently an aryl group substituted with one or more fluoro or fluoroalkyl, groups.

17. The holographic recording medium of Claim 16, wherein Ar₁ is a phenyl group substituted with one or more fluoro or fluoroalkyl groups, such as a phenyl group that is perfluorinated or substituted with one or more perfluoroalkyl groups, and Ar₄ is a phenyl group substituted with one or more fluoro or fluoroalkyl groups.

18. The holographic recording medium of Claim 17, wherein Ar₄-Ar₇, are independently perfluorinated or 3,5-bis(trifluoromethyl) substituted phenyl groups.

19. The holographic recording medium of Claim 18, wherein Ar₂-Ar₃ are independently substituted or unsubstituted phenyl groups.

20. The holographic recording medium of Claim 19, wherein the phenyl group represented by Ar₁ is perfluorinated, 3-trifluoromethyl substituted or 3,5-bis (trifluoromethyl) substituted.

21. The holographic recording medium of Claim 11, wherein the sulfonium salt is represented by the Structural Formula (II), as defined in Claim 7.

22. The holographic recording medium of Claim 21, wherein the monomer is an epoxide monomer.

23. The holographic recording medium of Claim 22, wherein the monomer comprises a cyclohexene oxide group.

24. The holographic recording medium of Claim 23, wherein the monomer is a siloxane comprising two or more cyclohexene oxide groups or a polyfunctional siloxane comprising three or more cyclohexene oxide groups.

25. The holographic recording medium of Claim 11, wherein the medium comprises a second monomer or oligomer which is capable of undergoing cationic polymerization.

26. The holographic recording medium of Claim 15 or the sulfonium salt of Claim 1, wherein Ar₁ and Ar₄-Ar₇ are further additionally substituted by substituents selected from halogens, (R)₃Si-, C₁-C₄ alkyl groups, or aryl groups, wherein each R is independently an unsubstituted aliphatic group or an unsubstituted aryl group

27. A method of recording holograms within a holographic recording medium of any of the Claims 11 to 26, comprising:
passing into said medium a reference beam of coherent actinic radiation and at substantially the same location in the medium simultaneously passing into the medium an object beam of the same coherent actinic radiation,
thereby forming within said medium an interference pattern and thereby recording a hologram within said medium.

28. A method of generating acid, comprising the step of exposing to visible light a composition comprising:
a) a sulfonium salt represented by the Structural Formula (V), as defined in Claim 8; and
b) a "photosensitizer", which in combination with the sulfonium salt produces acid at a particular wavelength of light, wherein the visible light has a wavelength greater than 500 nm.

## Patentansprüche

1. Sulfoniumsalz der Strukturformel (I): 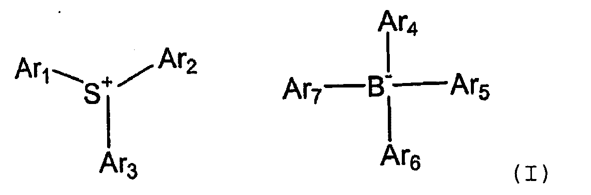 worin Ar₁ für eine Arylgruppe, die mit einer oder mehreren Fluoralkyl- oder Chlorgruppen substituiert ist, steht und Ar₂ - Ar₃ unabhängig voneinander für eine unsubstituierte Arylgruppe oder eine Arylgruppe, die mit Fluor, Chlor, Brom, Alkyl, Fluoralkyl, Chloralkyl, Bromalkyl, Perfluoralkyl oder Perchloralkyl substituiert ist, stehen und Ar₄ - Ar₇ unabhängig voneinander für eine unsubstituierte Arylgruppe oder eine Arylgruppe, die mit einer oder mehreren Fluor- oder Fluoralkylgruppen substituiert ist, stehen.

2. Sulfoniumsalz nach Anspruch 1, wobei Ar₄ - Ar₇ unabhängig voneinander für eine Arylgruppe, die mit einer oder mehreren Fluor- oder Fluoralkylgruppen substituiert ist, stehen.

3. Sulfoniumsalz nach Anspruch 1, wobei Ar₁ für eine Phenylgruppe, die mit einer oder mehreren Fluoralkyl- oder Perfluoralkylgruppen substituiert ist, steht und Ar₄ für eine Phenylgruppe, die mit einer oder mehreren Fluor- oder Fluoralkylgruppen substituiert ist, steht.

4. Sulfoniumsalz nach Anspruch 3, wobei Ar₄ - Ar₇ unabhängig voneinander für eine Phenylgruppe, die mit einer oder mehreren Fluor- oder Fluoralkylgruppen substituiert ist, oder perfluorierte oder 3,5-bis(trifluormethyl)-substituierte Phenylgruppen stehen.

5. Sulfoniumsalz nach Anspruch 4, wobei Ar₂ - Ar₃ unabhängig voneinander für substituierte oder unsubstituierte Phenylgruppen stehen.

6. Sulfoniumsalz nach Anspruch 5, wobei die durch Ar₁ dargestellte Phenylgruppe 3-trifluormethyl-substituiert oder 3,5-bis(trifluormethyl)-substituiert ist.

7. Sulfoniumsalz nach Anspruch 1, der Strukturformel (II): 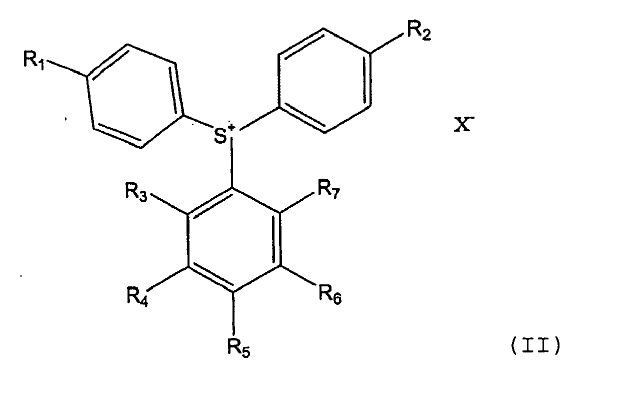 worin:
R₁ und R₂ für Methyl stehen; R₃ und R₅ - R₇ für -H stehen; und R₄ für -CF₃ oder -Cl steht;
R₁ - R₃ und R₅ - R₇ für -H stehen; und R₄ für -CF₃ oder -Cl steht;
R₁ und R₂ für Methyl stehen; R₃ und R₅ und R₇ für -H stehen; und R₄ und R₆ für -CF₃ stehen; oder
R₁ - R₃, R₅ und R₇ für -H stehen; und R₄ und R₆ für -CF₃ stehen; und
X⁻ durch die Strukturformel (III) oder (IV) dargestellt wird: 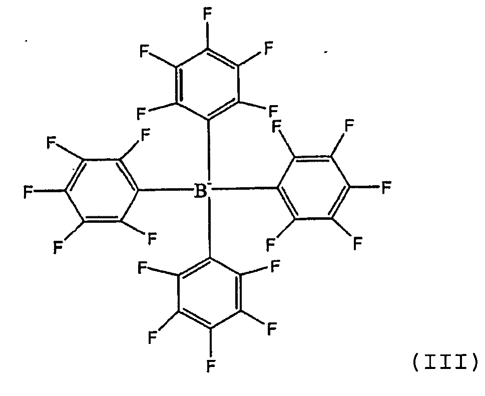 oder

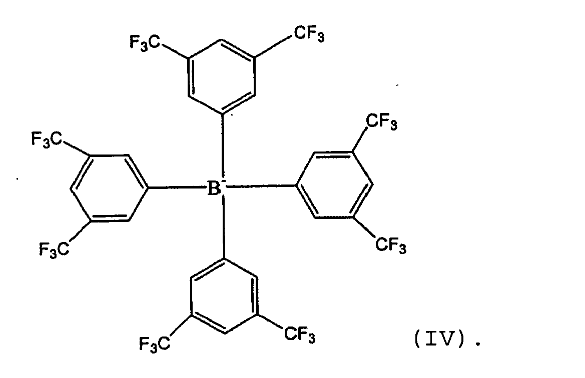

8. Polymerisierbares Medium, wobei das Medium umfasst:
a) ein Sulfoniumsalz der Strukturformel (V): 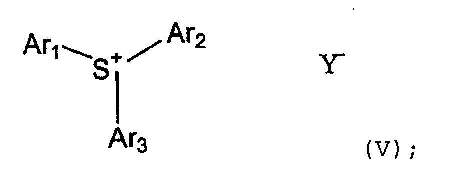 worin:
Ar₁ für eine Arylgruppe, die mit einer oder mehreren Fluoralkyl-, Fluor- oder Chlorgruppen substituiert ist, steht;
Ar₂ - Ar₃ unabhängig voneinander für eine unsubstituierte Arylgruppe oder eine Arylgruppe, die mit Fluor, Chlor, Brom, Alkyl, Fluoralkyl, Chloralkyl, Bromalkyl, Perfluoralkyl oder Perchloralkyl substituiert ist, stehen;
Y⁻ ausgewählt ist aus der Gruppe von B(R₈)ₓ(Ar₈)_{y}⁻, BF₄⁻, PF₆⁻, AsF₆⁻, SbF₆⁻, (CF₃SO₂)₃C⁻, (CF₃SO₂)₂N⁻, CF₃SO₃⁻, Ga (C₆F₅)₄⁻ und Carboranen;
jedes R₈ unabhängig voneinander für eine unsubstituierte Alkylgruppe oder eine Alkylgruppe, die mit einem Halogen, (R)₃Si-, einer Alkylgruppe oder einer Arylgruppe substituiert ist, steht, wobei jedes R unabhängig voneinander für eine unsubstituierte aliphatische Gruppe oder eine unsubstituierte Arylgruppe steht;
jedes Ar₈ unabhängig voneinander für eine unsubstituierte Arylgruppe oder eine Arylgruppe, die mit einem Halogen, (R)₃Si-, einer Alkylgruppe oder einer Arylgruppe substituiert ist, steht, wobei jedes R unabhängig voneinander für eine unsubstituierte aliphatische Gruppe oder eine unsubstituierte Arylgruppe steht; und
x und y für 0, 1, 2, 3 oder 4 stehen, wobei die Summe von x und y 4 ist;
b) einen "Photosensibilisator", der das Sulfoniumsalz als Reaktion auf sichtbares Licht mit einer Wellenlänge von größer als 500 nm zur Bildung einer Säure sensibilisiert; und
c) mindestens ein Monomer oder Oligomer, an dem eine kationische Polymerisation durchgeführt werden kann, die durch die ausgehend von dem Sulfoniumsalz gebildete Säure gestartet wird.

9. Polymerisierbares Medium nach Anspruch 8, wobei das Monomer oder Oligomer, an dem eine kationische Polymerisation durchgeführt werden kann, eine oder mehrere funktionelle Gruppen eines Epoxids, Oxetans, 1-Alkenylethers, cyclischen Ethers, Vinylethers, ungesättigten Kohlenwasserstoffs, Lactons, cyclischen Esters, Lactams, cyclischen Carbonats, cyclischen Acetals, Aldehyds, cyclischen Sulfids oder Cyclosiloxans oder eine Kombination derselben enthält.

10. Polymerisierbares Medium nach Anspruch 8, wobei das Medium ein Bindemittel umfasst.

11. Holographisches Aufzeichnungsmedium, wobei das Medium umfasst:
a) ein Sulfoniumsalz der Strukturformel (V) gemäß der Definition in Anspruch 8;
b) einen "Photosensibilisator", der in Kombination mit dem Sulfoniumsalz als Reaktion auf sichtbares Licht eine Säure bildet;
c) ein Monomer oder Oligomer, an dem eine kationische Polymerisation durchgeführt werden kann, die durch die Säure gestartet wird; und
d) ein Bindemittel, das zur Unterstützung der kationischen Polymerisation des Monomers oder Oligomers fähig ist.

12. Holographisches Aufzeichnungsmedium nach Anspruch 11, wobei der Photosensibilisator in Kombination mit dem Sulfoniumsalz als Reaktion auf sichtbares Licht mit einer Wellenlänge von größer als 500 nm und kürzer als 750 nm eine Säure bildet.

13. Holographisches Aufzeichnungsmedium nach Anspruch 11, wobei das Bindemittel diffusionsfähig und gegenüber Polymerisation inert ist.

14. Holographisches Aufzeichnungsmedium nach Anspruch 11, wobei Ar₁ für eine Arylgruppe, die mit einer oder mehreren Fluoralkyl- oder Fluorgruppen substituiert ist, beispielsweise eine Arylgruppe, die perfluoriert oder mit einer oder mehreren Perfluoralkylgruppen substituiert ist, steht.

15. Holographisches Aufzeichnungsmedium nach Anspruch 14, wobei durch die Formel B(Ar₄) (Ar₅) (Ar₆) (Ar₇)- dargestellt wird, worin Ar₄ - Ar₇ unabhängig voneinander für eine unsubstituierte Arylgruppe oder eine Arylgruppe, die mit einer oder mehreren Fluor- oder Fluoralkylgruppen substituiert ist, stehen.

16. Holographisches Aufzeichnungsmedium nach Anspruch 15, wobei Ar₄ - Ar₇ unabhängig voneinander für eine Arylgruppe, die mit einer oder mehreren Fluor- oder Fluoralkylgruppen substituiert ist, stehen.

17. Holographisches Aufzeichnungsmedium nach Anspruch 16, wobei Ar₁für eine Phenylgruppe, die mit einer oder mehreren Fluor- oder Fluoralkylgruppen substituiert ist, beispielsweise eine Phenylgruppe, die perfluoriert oder mit einer oder mehreren Perfluoralkylgruppen substituiert ist, steht und Ar₄ für eine Phenylgruppe, die mit einer oder mehreren Fluor-oder Fluoralkylgruppen substituiert ist, steht.

18. Holographisches Aufzeichnungsmedium nach Anspruch 17, wobei Ar₄ - Ar₇ unabhängig voneinander für perfluorierte oder 3,5-bis(trifluormethyl)-substituierte Phenylgruppen stehen.

19. Holographisches Aufzeichnungsmedium nach Anspruch 18, wobei Ar₂ - Ar₃ unabhängig voneinander für substituierte oder unsubstituierte Phenylgruppen stehen.

20. Holographisches Aufzeichnungsmedium nach Anspruch 19, wobei die durch Ar₁ dargestellte Phenylgruppe perfluoriert, 3-trifluormethyl-substituiert oder 3,5-bis(trifluormethyl)-substituiert ist.

21. Holographisches Aufzeichnungsmedium nach Anspruch 11, wobei das Sulfoniumsalz durch die Strukturformel (II) gemäß der Definition in Anspruch 7 dargestellt wird.

22. Holographisches Aufzeichnungsmedium nach Anspruch 21, wobei das Monomer ein Epoxidmonomer ist.

23. Holographisches Aufzeichnungsmedium nach Anspruch 22, wobei das Monomer eine Cyclohexenoxidgruppe umfasst.

24. Holographisches Aufzeichnungsmedium nach Anspruch 23, wobei das Monomer ein Siloxan, das zwei oder mehr Cyclohexenoxidgruppen umfasst, oder ein polyfunktionales Siloxan, das drei oder mehr Cyclohexenoxidgruppen umfasst, ist.

25. Holographisches Aufzeichnungsmedium nach Anspruch 11, wobei das Medium ein zweites Monomer oder Oligomer, an dem eine kationische Polymerisation durchgeführt werden kann, umfasst.

26. Holographisches Aufzeichnungsmedium nach Anspruch 15 oder Sulfoniumsalz nach Anspruch 1, wobei Ar₁und Ar₄ - Ar₇ ferner zusätzlich durch Substituenten, die aus Halogenen, (R)₃Si-, C₁-₄-Alkylgruppen oder Arylgruppen ausgewählt sind, substituiert sind, wobei jedes R unabhängig voneinander für eine unsubstituierte aliphatische Gruppe oder eine unsubstituierte Arylgruppe steht.

27. Verfahren zur Aufzeichnung von Hologrammen in einem holographischen Aufzeichnungsmedium nach einem der Ansprüche 11 bis 26, umfassend:
das Hineinführen eines Referenzstrahls einer kohärenten photochemisch wirksamen Strahlung in das Medium und, an der im Wesentlichen gleichen Stelle in dem Medium, das gleichzeitige Hineinführen eines Objektstrahls der gleichen kohärenten photochemisch wirksamen Strahlung in das Medium,
wodurch in dem Medium ein Interferenzmuster ausgebildet wird und **dadurch** ein Hologramm in dem Medium aufgezeichnet wird.

28. Verfahren zur Bildung einer Säure, umfassend die Stufen des Einwirkens von sichtbarem Licht auf eine Zusammenfassung, die umfasst:
a) ein Sulfoniumsalz der Strukturformel (V) gemäß der Definition in Anspruch 8 und
b) einen "Photosensibilisator", der in Kombination mit dem Sulfoniumsalz bei einer speziellen Lichtwellenlänge eine Säure bildet, wobei das sichtbare Licht eine Wellenlänge von größer als 500 nm aufweist.

## Revendications

1. Sel de sulfonium représenté par la formule structurale (I) : 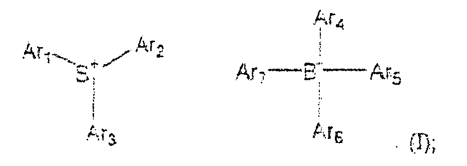 dans laquelle Ar₁ est un groupe aryle substitué par un ou plusieurs groupes fluoroalkyles ou chloro et Ar₂-Ar₃ sont indépendamment un groupe aryle non substitué ou un groupe aryle substitué par un fluoro, un chloro, un bromo, un alkyle, un fluoroalkyle, un chloroalkyle, un bromoalkyle, un perfluoroalkyle ou un perchloroalkyle, et Ar₄ - Ar₇ sont indépendamment un groupe aryle non substitué ou un groupe aryle substitué par un ou plusieurs groupes fluoro ou fluoroalkyles.

2. Sel de sulfonium selon la revendication 1, dans lequel Ar₄ - Ar₇ sont indépendamment un groupe aryle substitué par un ou plusieurs groupes fluoro ou fluoroalkyles.

3. Sel de sulfonium selon la revendication 1, dans lequel Ar₁ est un groupe phényle substitué par un ou plusieurs groupes fluoroalkyles ou perfluoroalkyles et Ar₄ est un groupe phényle substitué par un ou plusieurs groupes fluoro ou fluoroalkyles.

4. Sel de sulfonium selon la revendication 3, dans lequel Ar₄ - Ar₇ sont indépendamment un groupe phényle substitué par un ou plusieurs groupes fluoro ou fluoroalkyles ou des groupes phényles perfluorés ou à substitution 3,5-bis(trifluorométhyle).

5. Sel de sulfonium selon la revendication 4, dans lequel Ar₂-Ar₃ sont indépendamment des groupes phényles substitués ou non substitués.

6. Sel de sulfonium selon la revendication 5, dans lequel le groupe phényle représenté par Ar₁ est à substitution 3-trifluorométhyle ou à substitution 3,5-bis(trifluorométhyle).

7. Sel de sulfonium selon la revendication 1, représenté par la formule structurale (II) : 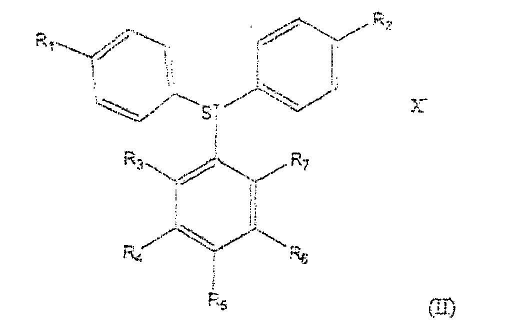 dans laquelle :
R₁et R₂ sont un méthyle ; R₃ et R₅ - R₇ sont -H ; et R₄ est -CF₃ ou -Cl;
R₁ - R₃ et R₅ - R₇ sont -H ; et R₄ est -CF₃ ou -Cl ;
R₁et R₂ sont un méthyle ; R₃, R₅ et R₇ sont -H ; et R₄ et R₆ sont -CF₃ ; ou
R₁ - R₃ , R₅ et R₇ sont -H ; et R₄ et R₆ sont -CF₃ ; et
X⁻ est représenté par la formule structurale (III) ou (IV) : 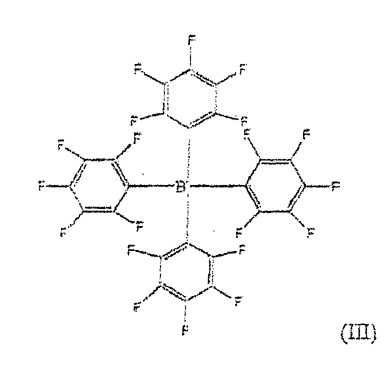 ou

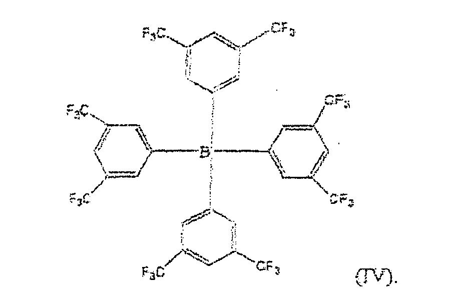

8. Milieu polymérisable, lequel milieu comprend :
a) un sel de sulfonium représenté par la formule structurale (V) : 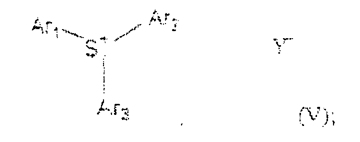 dans laquelle :
Ar₁ et un groupe aryle substitué par un ou plusieurs groupes fluoroalkyles, fluoro ou chloro ;
Ar₂-Ar₃ sont indépendamment un groupe aryle non substitué ou un groupe aryle substitué par un fluoro, un chloro, un bromo, un alkyle, un fluoroalkyle, un chloroalkyle, un bromoalkyle, un perfluoroalkyle ou un perchloroalkyle ;
Y⁻ est choisi dans le groupe constitué par B(R₈)ₓ(Ar₈)_{y}⁻, BF₄⁻, PF₆⁻, AsF₆⁻, SbF₆⁻, (CF₃SO₂)₃C⁻, (CF₃SO₂N⁻, CF₃SO₃⁻, Ga (C₆F₅)₄⁻ et les carboranes ;
chaque R₈ est indépendamment un groupe alkyle non substitué ou un groupe alkyle substitué par un halogène, (R)₃Si-, un groupe alkyle ou un groupe aryle, où chaque R est indépendamment un groupe aliphatique non substitué ou un groupe aryle non substitué ;
chaque Ar₈ est indépendamment un groupe aryle non substitué ou un groupe aryle substitué par un halogène, (R)₃Si-, un groupe alkyle ou un groupe aryle, où chaque R est indépendamment un groupe aliphatique non substitué ou un groupe aryle non substitué ; et
x et y sont 0, 1, 2, 3 ou 4, où la somme de x et y est 4 ;
b) un "photosensibilisateur", qui sensibilise le sel de sulfonium à produire un acide en réponse à une lumière visible ayant une longueur d'onde supérieure à 500 nm ; et
c) au moins un monomère ou oligomère qui est apte à subir une polymérisation cationique initiée par l'acide produit par le sel de sulfonium.

9. Milieu polymérisable selon la revendication 8, dans lequel le monomère ou l'oligomère qui est apte à subir une polymérisation cationique contient un ou plusieurs groupes époxydes, oxétanes, éthers 1-alcényliques, éthers cycliques, éthers vinyliques, hydrocarbonés insaturés, lactones, esters cycliques, lactames, carbonates cycliques, acétals cycliques, aldéhydes, sulfures cycliques ou fonctionnels cyclosiloxanes, ou une combinaison de ceux-ci.

10. Milieu polymérisable selon la revendication 8, lequel milieu comprend un liant.

11. Milieu d'enregistrement holographique, lequel milieu comprend :
a) un sel de sulfonium représenté par la formule structurale (V), telle que définie dans la revendication 8 ;
b) un "photosensibilisateur" qui, en combinaison avec le sel de sulfonium, produit un acide en réponse à une lumière visible ;
c) un monomère ou oligomère qui est apte à subir une polymérisation cationique déclenchée par l'acide ; et
d) un liant qui est apte à supporter la polymérisation cationique dudit monomère ou oligomère.

12. Milieu d'enregistrement holographique selon la revendication 11, dans lequel le photosensibilisateur, en combinaison avec le sel de sulfonium, produit un acide en réponse à une lumière visible ayant une longueur d'onde supérieure à 500 nm et plus courte que 750 nm.

13. Milieu d'enregistrement holographique selon la revendication 11, dans lequel le liant est diffusible et inerte vis-à-vis d'une polymérisation.

14. Milieu d'enregistrement holographique selon la revendication 11, dans lequel Ar₁et un groupe aryle substitué par un ou plusieurs groupes fluoroalkyles ou fluoro, comme un groupe aryle qui est perfluoré ou substitué par un ou plusieurs groupes perfluoroalkyles.

15. Milieu d'enregistrement holographique selon la revendication 14, dans lequel Y est représenté par la formule B(Ar₄) (Ar₅) (Ar₆) (Ar₇)⁻, où Ar₄ - Ar₇ sont indépendamment un groupe aryle non substitué ou un groupe aryle substitué par un ou plusieurs groupes fluoro ou fluoroalkyles.

16. Milieu d'enregistrement holographique selon la revendication 15, dans lequel Ar₄ - Ar₇ sont indépendamment un groupe aryle substitué par un ou plusieurs groupes fluoro ou fluoroalkyles.

17. Milieu d'enregistrement holographique selon la revendication 16, dans lequel Ar₁ est un groupe phényle substitué par un ou plusieurs groupes fluoro ou fluoroalkyles, comme un groupe phényle qui est perfluoré ou substitué par un ou plusieurs groupes perfluoroalkyles, et Ar₄ est un groupe phényle substitué par un ou plusieurs groupes fluoro ou fluoroalkyles.

18. Milieu d'enregistrement holographique selon la revendication 17, dans lequel Ar₄ - Ar₇ sont indépendamment des groupes phényles perfluorés ou à substitution 3,5-bis(trifluorométhyle).

19. Milieu d'enregistrement holographique selon la revendication 18, dans lequel Ar₂-Ar₃ sont indépendamment des groupes phényles substitués ou non substitués.

20. Milieu d'enregistrement holographique selon la revendication 19, dans lequel le groupe phényle représenté par Ar₁ est perfluoré, à substitution 3-trifluorométhyle ou à substitution 3,5-bis(trifluorométhyle).

21. Milieu d'enregistrement holographique selon la revendication 11, dans lequel le sel de sulfonium est représenté par la formule structurale (II), telle que définie dans la revendication 7.

22. Milieu d'enregistrement holographique selon la revendication 21, dans lequel le monomère est un monomère époxyde.

23. Milieu d'enregistrement holographique selon la revendication 22, dans lequel le monomère comprend un groupe oxyde de cyclohexène.

24. Milieu d'enregistrement holographique selon la revendication 23, dans lequel le monomère est un siloxane comprenant deux groupes oxyde de cyclohexène ou plus, ou un siloxane polyfonctionnel comprenant trois groupes oxyde de cyclohexène ou plus.

25. Milieu d'enregistrement holographique selon la revendication 11, lequel milieu comprend un second monomère ou oligomère qui est apte à subir une polymérisation cationique.

26. Milieu d'enregistrement holographique selon la revendication 15 ou sel de sulfonium selon la revendication 1, dans lequel Ar₁ et Ar₄ - Ar₇ sont en outre supplémentairement substitués par des substituants choisis parmi les halogènes, (R)₃Si-, les groupes alkyles en C₁-C₄, ou les groupes aryles, où chaque R est indépendamment un groupe aliphatique non substitué ou un groupe aryle non substitué.

27. Procédé d'enregistrement d'hologrammes à l'intérieur d'un milieu d'enregistrement holographique selon l'une quelconque des revendications 11 à 26, comprenant :
le passage dans ledit milieu d'un faisceau de référence de radiation actinique cohérente et, essentiellement au même endroit dans le milieu, le passage simultanément dans le milieu d'un faisceau objet de la même radiation actinique cohérente ;
la formation ainsi, à l'intérieur dudit milieu, d'un motif d'interférence et à enregistrer ainsi un hologramme à l'intérieur dudit milieu.

28. Procédé de génération d'acide, comprenant l'étape consistant à exposer à une lumière visible une composition comprenant :
a) un sel de sulfonium représenté par la formule structurale (V), telle que définie dans la revendication 8 ; et
b) un "photosensibilisateur" qui, en combinaison avec le sel de sulfonium, produit un acide à une longueur d'onde de lumière particulière, la lumière visible ayant une longueur d'onde supérieure à 500 nm.
